# EUROPEAN PATENT SPECIFICATION

(11) **EP 4 014 936 B1**
(45) Date of publication and mention of the grant of the patent: **07.08.2024**
(21) Application number: 20215666.7
(22) Date of filing: 18.12.2020
(51) Int. Cl.: A61F 13/42

(54) **IMPROVED SENSOR SYSTEM FOR DETECTING A PROPERTY OF OR WITHIN AN ABSORBENT ARTICLE AND ABSORBENT ARTICLE COMPRISING SUCH SENSOR SYSTEM**
VERBESSERTES SENSORSYSTEM ZUR ERFASSUNG EINER EIGENSCHAFT VON ODER IN EINEM ABSORBIERENDEN ARTIKEL UND ABSORBIERENDER ARTIKEL MIT EINEM SOLCHEN SENSORSYSTEM
SYSTÈME DE CAPTEUR AMÉLIORÉ POUR LA DÉTECTION D'UNE PROPRIÉTÉ DE OU DANS UN ARTICLE ABSORBANT ET ARTICLE ABSORBANT COMPRENANT UN TEL SYSTÈME DE CAPTEUR

(43) Date of publication of application: 22.06.2022
(73) Proprietor: Ontex BV, 9255 Buggenhout (BE); Ontex Group NV, 9320 Erembodegem (BE)
(72) Inventor: WEBER, Ainas, 53474 Bad Neuenahr-Ahrweiler (DE)
(74) Representative: Saurat, Thibault

(56) References cited:
- WO-A1-2018/229017
- US-A- 5 416 469
- US-A1- 2012 165 770
- US-A1- 2015 356 852
- US-A1- 2019 365 572
- US-B2- 10 357 409

## Description

### TECHNICAL FIELD

The invention pertains to the technical field of absorbent hygiene products. In particular, the present invention relates to an absorbent article for absorbing body fluids and exudates, such as urine and fecal material. More particularly, the present invention relates to absorbent garments, such as disposable diapers or pants for example for babies or adults, which are configured to collect and contain fecal material and avoid leakage.

Most particularly the invention pertains to diapers or pants, components thereof, comprising an improved exudate detection system capable of automatically providing a warning to a care giver when the risk of leakage is elevated and the diaper or pant warn by a subject should be replaced.

### BACKGROUND

Disposable diapers conventionally include a chassis having a liquid permeable topsheet, a liquid impermeable backsheet and an absorbent structure sandwiched between the topsheet and backsheet. The chassis has a front body panel which, in use, extends over the stomach and front hip area of the user, and a rear body panel which, in use, extends over the back and the rear hip area of the user. Each of the body panels has a waist portion such that, when the diaper is fastened around the waist of the user, the waist portions provide a continuous encirclement of the user. In order to fasten the diaper around the waist of a user, a fastening system comprising fastening tabs is commonly employed. Fastening tabs may be provided on side panels which extend from lateral side edges of the diaper chassis.

Disposable pants have a similar construction but typically comprise front and back elasticized belts at either end of the absorbent structure and are sealed together at lateral side seams to form an underwear-resembling product that can be worn by a subject by pulling it up over the legs and may be removed either by pulling it down in the opposite direction or by tearing the side seams.

Inherent with the use of such absorbent articles, eventually one or more wetness and/or exudate events will occur and thus a need for change of the absorbent article arises. Particularly in home care institutions handling elderly with incontinence problems, knowing when to change the diaper/pant of a patient is important. Indeed, changing the diapers too early results in unnecessary cost and waste and changing too late results in further cleaning costs and time, and uncomfort to the patient. This problem is exacerbated by motion, indeed taking into account that a subject wearing the article may move into different positions the saturation thereof and/or risk of leakage may vary considerably based on the position and time the subject remains in that position during and/or after one or more wetness/exudate events.

Absorbent articles possessing different types of detecting means are known, and help to alert a user or caregiver to a change within the article (e.g. a soiling event). Such detecting means allow the user or caregiver to readily determine whether or not an absorbent article needs to be changed, without the need for close inspection or removal of the article. Commonly known detecting means which can be incorporated into absorbent articles are chemical substances which alter their form or nature upon contact with liquid. For example, an indication that an absorbent article is soiled can arise from colour changes or the appearance or disappearance of an element on the absorbent article. Such technology is known from, e.g. US 5 389 093 , WO 04/028403 and WO 05/030084. Such detecting means are useful in certain situations, but less so in institutions such as childcare centres, care centres for the elderly or hospitals where the status of a large number of wearers must be monitored, often by a limited staff. Determining whether the absorbent article is soiled or not still requires the wearer to be disturbed, as the coloured element must still be visible to the caregiver. This often requires that the wearer be moved, and their clothes removed or adjusted.

WO02/47592 describes an article having a status signalling device for communicating a change in status of a monitored portion. The signalling device can comprise a sensor located within the article, the sensor being connected to an external portion located on the outside of the article. Changes in the status of the article (e.g. soiling) can be transmitted from the signalling device to a receiver via an RF link produced by the external portion.

The external portion is included on the outside of the article and is secured in place, e.g. by hook-and-loop type fasteners. As such, it can be removed or displaced and is subject to external influences (e.g. abrasion, moisture, interference by the wearer). Furthermore, traditional components of the external portion described in WO02/47592 render it comparatively expensive to produce, which in turn, renders its disposal expensive and reuse more likely.

US 2005/0156744 describes a diaper similar to that of WO02/47592 , in which a detachable transmitter is installed on the outside of the diaper.

WO02/78513 describes a fluid discharge monitoring apparatus for a diaper. The apparatus comprises an RF tag which is responsive to the discharge of fluid into the diaper. There is no discussion in WO02/78513 as to the nature of the components which are used in the fabrication of the monitoring apparatus.

JP 2005000602 describes a wet detecting device in a diaper, comprising an RF-ID tag. The tag comprises an IC chip, a communication control section, data storage medium and an antenna.

WO 99/33037 discloses a method and apparatus for detecting a fluid, said method comprising providing one or more oscillators transmitting electromagnetic energy, providing one or more resonant circuits receiving electromagnetic energy from the oscillators, bringing the fluid and the one or more resonant circuits into contact with each other so that the receptions of the electro-magnetic energy of the resonant circuits are changed, and detecting changes of the transmissions of electromagnetic energy of the oscillators by changes in one or more characteristics thereof upon the changes in the receptions of the electromagnetic energy of the resonant circuits. The resonant circuits may consist of coils having separated windings made of an electrically conducting material, which may be provided by printing on a substrate. The resonant circuit(s) may be embedded in a diaper. Thus, WO 99/33037 discloses an absorbent article comprising at least one wetness detecting means.

Although electrical detecting means for indicating the status of an absorbent article have clear advantages over visual detecting means, they still suffer from the drawbacks of being expensive, stiff, bulky and difficult to incorporate into the article during manufacture. In addition, many traditional electrical components are not readily disposable or degradable. Traditional components of electrical circuits such as soldered metal are not compatible with materials such as paper, plastics and fibrous materials used in modern absorbent articles. Neither are they compatible with the rapid assembly-line manufacturing methods used in the production of absorbent articles. As absorbent articles are primarily intended for single use (i.e. they are disposable), it would be a great advantage if electrical detecting means were cheap and readily disposable. There is thus a desire for a detecting means which can be readily incorporated into an absorbent article which provides the advantages of electrical detection, yet which is cost-effective, simple to manufacture and readily disposable.

To that extent, new detection systems emerged such as in US20130233063 or EP3415130 that integrates micro-electronic components which are more suitable for providing accurate and reliable sensing and that can be standardized across a range of product and sizes in order to limit production cost and thus the end cost to the users and institutions.

However, these systems have several drawbacks. The foremost problem is these systems do not take into account the factor of time, in other words the evolution of a physical parameter throughout time to better assess the condition of or within the absorbent article. In other words, it is complicated to have a proper reading throughout time which can cause a care giver to change a diaper too often or not often enough. Another issue is that such micro-electronic devices are energy-consuming and can deplete a battery fast, these systems are meant to be on a absorbent article which means that charging by a electric wall-outlet is not conceivable. Another issue is that is that these system integrate only one kind of sensor which means that the assessment of the condition of the absorbent article is going to be based on one physical parameter. This causes a less precise interpretation.

Document US2019/0365572 discloses a system comprising a plurality of sensors disposed in an infant product and document US10,357,409 describes a signalling device 110 for an absorbent article including a plurality of capacitive sensors however these systems still have an elevated power consumption.

There remains a need for an improved sensor system enabling a more accurate reading that can take into account the evolution of the condition of or within the absorbent article through time and that is less energy-consuming.

The invention thereto aims to provide an improved sensor system for detecting a property of or within an absorbent article and absorbent article comprising such sensor system.

### SUMMARY OF THE INVENTION

The present invention provides a sensor system configured for detecting a property of or within an absorbent article, comprising:
- a first sensor configured to measure a first physical parameter, said first sensor being configured to detect a change in condition of or within the absorbent article;
- a second sensor configured to measure a second physical parameter, said second sensor being configured to detect a change in condition of or within the absorbent article,
wherein the first and second physical parameters are different from one another. According to the invention, the first sensor is configured to measure electrical resistance or electrical conductance and the second sensor is configured to measure temperature and wherein the second sensor is a multispectral and/or thermal imaging camera.

The term "physical parameter" used herein refers to a measurable physical characteristic of or within the absorbent article, including, but not limited to, temperature, electrical conductance, electrical resistance, capacitance, pH, mass, solubility, pressure, length, volumetric flow, positioning within a space (or area). Therefore the first sensor can measure a first physical characteristic, for example and not limited to, temperature while the second sensor can measure a different physical characteristic, for example and not limited to, electrical resistance.

The terms "property" or "condition" used herein refer to the physical state of or within the absorbent article including but not limited to, the level of dryness, meaning the presence or lack of urine, the presence or lack of fecal matter, gush, blood, or any other bodily fluids. The property or condition of or within the absorbent article alters following an expulsion of urine, fecal matter or other bodily fluids, such expulsion called wetness, soilage, exudate and/or spill event herein.

The sensor system according to the invention enables to measure two different physical characteristics of or within the absorbent article. A physical characteristic has its own evolution throughout time, thereby by monitoring two distinct physical parameters that each evolves differently throughout time, it is possible to have a better reading of the status of or within the absorbent article.

According to an embodiment, the second sensor is distinct and remote from the first sensor.

These two physical parameters have different evolution throughout time and the monitoring of these two physical parameters is particularly adapted for this sensor system. Electrical resistance, or electrical conductance, enables to quantify the level of wetness of or within the absorbent article but has little variation throughout time once a first wetness/soiling/exudate/spill event has passed, whereas temperature enables to detect precisely the occurrence of each wetness/soiling/exudate/spill event but gives less information as to the amount of bodily fluid expulsed during a wetness/soiling/exudate/spill event.

A thermal camera is well adapted to measure temperature from afar. Furthermore, a multispectral and/or thermal imaging camera enables to monitor several patients within an area.

According to an embodiment, the second sensor is linked to a temperature probe.

According to an embodiment, the sensor system comprises a third sensor configured to measure a third physical parameter, said third sensor being configured to detect a change in condition of or within the absorbent article; wherein the first, second and third physical parameters are different from one another.

According to an embodiment, the sensor system monitors the temperature and electrical resistance, or electrical conductance, behaviour of or within the absorbent article by measuring the temperature and electrical resistance or electrical conductance values and plotting said values against time.

According to an embodiment, the first sensor is activated directly or indirectly by the second sensor.

As explained above, the electrical resistance, or electrical conductance, sensor is power consuming, whereas the temperature sensor requires less energy to run on. Therefore according to this embodiment, once the second sensor detects a rise in temperature indicating a wetness event, it can send an order to activate the electrical resistance or electrical conductance sensor to measure the amount of bodily fluid. The electrical resistance, or electrical conductance, sensor can be on stand-by until activated by the temperature sensor thus saving battery power. This embodiment is particularly adapted when the second sensor is a multispectral and/or thermal imaging camera.

According to an embodiment, the sensor system further comprises a control system that manages the output data of first, second and/or third sensors, said control system further comprises a user interface that manages the output data of the control system.

The user interface may correspond to a computer and its screen or any device that can display information including but not limited to a phone and its screen, a tablet and its screen, so as to be easily viewed by a care giver. This user interface can emit alerts, give stationary status.

According to an embodiment, the control system manages the output data of the second sensor, i.e. the temperature values, and if a spike in temperature is detected, the control system activates the first sensor in order to measure electrical resistance, or electrical conductance, of or within the absorbent article.

In the case where the second sensor is a multispectral and/or thermal imaging camera, there may be an additional prior step in which camera image corresponds to an array of multiple pixels with each pixel carrying a temperature value thus detecting a "spike" would then require an algorithm to identify if a cluster of pixels with elevated temperature is considered a "spike" event or not.

The invention also covers an assembly of an absorbent article and a sensor system as described as above.

According to an embodiment, the first sensor and/or second sensor are disposed in or on the absorbent article, and wherein the first and/or second sensor are separable from the absorbent article.

According to an embodiment, the absorbent article comprises a substrate suitable for incorporation into an absorbent article for automatic detection of wetness events therein and/or risk of exudate leakage therefrom, the substrate comprising a first surface capable of being arranged proximal to a body facing side of the absorbent article and a second surface opposite said first surface and capable of being arranged proximal to a garment facing side of said absorbent article, said substrate comprising a plurality of sensor tracks disposed on said first surface and said sensor tracks comprising: at least one central track extending parallel to a length L of the substrate and parallel to a longitudinal axis (y-y) crossing a first end and a second end of the substrate; at least one or more side tracks extending parallel to the central track and oppositely arranged such that the central track extends therebetween; and wetness sensing tracks extending outboard of said two side tracks, wherein said central track, said side tracks, and said wetness sensing tracks are in electrical communication via one or more shortening elements positioned proximal to said second end and distal from said first end, and wherein the substrate is connectable to a clip-on data processing module at a position proximal to said first end and distal from said shortening elements such to form a closed electrical circuit, typically for measuring electrical resistance, electrical conductance, impedance and/or capacitance therethrough.

According to an embodiment, said substrate further comprises a plurality of thermal sensor tracks disposed on said second surface and said thermal sensor tracks comprising: at least one central thermal sensor track extending parallel to a length L of the substrate and parallel to a longitudinal axis (y-y) crossing a first end and a second end of the substrate; and wherein the substrate is connectable to a clip-on data processing module at a position proximal to said first end for measuring temperature therethrough.

According to an embodiment, said thermal sensor tracks are disposed on the first surface of the substrate.

According to an embodiment, the absorbent article further comprising a removable clip-on data processing module adapted to monitor and process electric resistance, electric conductance, and/or capacitance and/or temperature data acquired from the wetness sensing tracks and/or the thermal sensor tracks, said clip-on data processing module being connectable to the at least one central track and the at least two side tracks via a slit and/or pocket on the backsheet enabling an electrically conductive portion of said module to directly come in electrical communication with said at least one central track and said at least one side tracks, preferably the slit and/or pocket arranged to accommodate at least 50% of the total surface area of the clip on data processing module therein.

According to an embodiment, the said substrate futher comprises a plurality of thermal sensor tracks disposed on said second surface and said thermal sensor tracks comprising: at least one thermal central track extending parallel to a length L of the substrate and parallel to a longitudinal axis crossing a first end and a second end of the substrate; and wherein the substrate is connectable to a clip-on data processing module at a position proximal to said first end for measuring temperature therethrough.

According to an embodiment, the clip-on data processing module comprises a housing having a U-shaped cross-section, the housing having two flexible connection member, the substrate extending between the flexible connection members, each flexible connection member comprising connection ports to establish electrical connections between the sensor tracks and/or thermal sensor tracks and the clip-on data processing module.

According to another embodiment, the absorbent article comprises a substrate suitable for incorporation into an absorbent article for monitoring and detecting the presence of wetness therein and/or risk of exudate leakage therefrom, comprises a conductive pattern disposed on a first surface capable of being arranged proximal to a body facing side of the absorbent article, wherein said conductive pattern can be brought in electrical communication with a clip-on data processing module, said conductive pattern comprising a plurality of connection tracks; and a plurality of sensing tracks connected to said connection tracks, characterized in that the conductive pattern is configured in a manner that addressing multiple combinations of said plurality of connection tracks with corresponding connection ports of the clip-on data processing module result in multiple electrical circuit configurations for measuring resistance, conductance, impedance and/or capacitance therethrough.

According to a another embodiment, the substrate comprises a second surface opposite said first surface and capable of being arranged proximal to a garment facing side of said absorbent article. In addition to this or as an alternative, said clip-on data processing module is in electrical communication with the conductive pattern when connected at a position proximal to a first end of the substrate such to form at least one closed electrical circuit. This is beneficial, since the implementation of the inventive substrate into the final product is compatible with current production lines, which reduces operation costs compared to other solutions that require additional more complicated process steps and, furthermore, enables the use of the final product in a harmless manner for a wearer.

According to another embodiment, said connection tracks are configured to allow an electrical current to flow from corresponding terminals of the clip-on data processing module when connected to it.

According to another aspect, the substrate further comprises at least one insulating layer preferably being liquid impermeable, adhered thereto and sized to cover at least a portion of the conductive pattern, said at least one insulating layer adapted to provide a seal and/or barrier to liquid and/or exudates from coming into contact with said portion of the conductive pattern, preferably wherein the sensing tracks remain exposed to liquids and/or exudates and are not covered by said at least one insulating layer. This is beneficial since an insulating layer, being an inherent component of the final product, contributes to define specific zones where wetness can be selectively detected, enhancing efficiency.

According to another aspect, said substrate comprises one or more slits, and a pocket is formed between the first surface and the at least one insulating layer proximal to the first end, said pocket being in fluid communication only with said slit(s) and arranged to retain at least a portion of the clip-on data processing module therein. Advantageously, this arrangement enables a secure electrical connection between the clip-on module and the connection tracks whilst ensuring also its protection from soiling by internal and/or external elements. Furthermore, comfort for the wearer is also enhanced.

According to another aspect, the conductive pattern extends symmetrically in a direction substantially parallel to the length of the substrate and a direction substantially perpendicular thereto, defining a plurality of independent electrical circuits, and wherein combinations of said electrical circuits are in electrical communication by selectively addressing at least two of the connection tracks with at least two corresponding terminals of the clip-on data processing module. Advantageously, this ensures that a reliable detection in several zones along the length and width of the final product will be monitored, enhancing efficiency and accuracy. Further advantageously, a direct relation of the monitored zones can be related to body position of the subject wearing the product without requiring using additional motion sensors and/or gyroscopes, increasing efficiency and saving time.

According to another aspect, the plurality of circuits defined by the conductive pattern are circuits with a closed configuration and/or alternatively with an open configuration. In addition to this or as an alternative, at least one of the plurality of circuits defined by the conductive pattern is a circuit with a closed configuration. Additionally or alternatively, the plurality of circuits defined by the conductive pattern have a configuration that enables space for applying glue lines when incorporated into an absorbent article. This is beneficial, since the electrical properties of different circuits are used to enhance sensitivity on detecting and monitoring wetness. Further advantageously, appropriate electrical connection is ensured.

According to a further preferred implementation, a thickness and/or a length and/or a width and/or a specific conductivity of at least a portion of at least one of the plurality of circuits defined by the conductive pattern comprises identifiers of the absorbent article that are detected when connected to the clip-on data processing module. Advantageously, this increases efficiency and reduces fabrication costs.

Preferably, the conductive pattern comprises at least three, preferably at least four, more preferably at least, most preferably between 7 and 15 connection tracks and/or sensing tracks. This allows for an especially accurate sensing of exudate levels.

According to another aspect, an absorbent article, preferably a disposable diaper, pad or pant, suitable for monitoring and detecting the presence of wetness therein and/or risk of exudate leakage therefrom, is provided. Said absorbent article comprising: a liquid impermeable backsheet; a liquid permeable topsheet; an absorbent core interposed between said backsheet and topsheet, wherein said backsheet comprises a substrate, the substrate comprising a conductive pattern disposed on a first surface and can be brought in electrical communication with a clip-on data processing module and optionally thermal sensor tracks, said conductive pattern comprising: a plurality of connection tracks; and a plurality of sensing tracks connected to said connection tracks, and wherein the conductive pattern is configured in a manner that addressing multiple combinations of said plurality of connection tracks with corresponding terminals of the clip-on data processing module result in multiple electrical circuit configurations for measuring resistance, impedance and/or capacitance therethrough. Advantageously, accurate and automatic detection and monitoring of liquids and/or exudates in an efficient manner is enabled, ensuring scalable in in-line mass production at high speeds.

According to another embodiment, said absorbent article further comprises a removable clip-on data processing module having a plurality of exposed electrically conductive terminals capable of selectively addressing a plurality of connection tracks of a conductive pattern, preferably wherein the number of said terminals is at least the same as the number of said connection tracks. This is beneficial since appropriate electrical connection of said clip-on module with the connection tracks is ensured, since the clip-on module remains secured to the absorbent article whilst being protected from accidental disengagement during movements of the subject/wearer.

According to an embodiment, selectively addressing multiple combinations of the plurality of said connection tracks with corresponding terminals of the clip-on data processing module result in multiple electrical circuit configurations for measuring resistance, impedance and/or capacitance therethrough. In addition to this or as an alternative, at least a portion of the clip-on data processing module is retained within a pocket, said pocket positioned over at least a portion of the absorbent core with the core being interposed between said pocket and the skin of a subject when the absorbent article is worn such that when the clip-on data processing module is inserted into the pocket, the absorbent core provides a cushioning layer between said clip-on data processing module and said skin of the subject.

Advantageously, this arrangement allows to selectively define various zones of the final product where it is desired to detect whether wetness due to liquids and/or exudates occur.

According to a further embodiment, measuring resistance, impedance and/or capacitance of multiple circuit configurations defined by the conductive pattern selectively in contact with corresponding terminals of the clip-on data processing module enables monitoring wetness at different zones of the core of the absorbent article and/or monitoring a distribution of wetness as a result of the actual and/or previous body position of a wearer of said absorbent article. Advantageously, this enables to effectively detect wetness and to generate a mapping of wetness with high accuracy, from which also body position of the wearer is indirectly inferred.

Advantageously, the sensing tracks are arranged so that a separate monitoring of at least three, preferably at least four, most preferably at least five different zones along a lengthwise direction of the core of the absorbent article is possible. This allows an especially fine accuracy of the monitoring.

Preferably, the sensing tracks are arranged so that a separate monitoring of at least two, preferably at least three, most preferably at least four different zones along a widthwise direction of the core of the absorbent article is possible. This further increases monitoring accuracy.

The disclosure can also pertain to a method for making an absorbent article as described above comprising the steps of:
providing a liquid impermeable backsheet and applying a conductive pattern, as described previously, and optionally thermal sensor tracks as described herein to a first surface thereof;
providing and adhering at least one insulating layer to said backsheet, said insulating layer being sized and positioned to cover at least a portion of the connection tracks of the conductive pattern, to provide a laminated substrate;
providing an absorbent core comprising absorbent material;
providing a liquid permeable topsheet; and
sandwiching the absorbent core between said laminated substrate and said topsheet.

Alternatively, the method for making an absorbent article as described above can also comprise the steps of:
providing a liquid impermeable backsheet and applying a plurality of sensor tracks and optionally thermal sensor tracks as described herein;
providing an insulating layer having a width w, taken along an axis perpendicular to the longitudinal axis y-y, being less than a width W of said backsheet, and typically applying one or more shortening elements thereto,
optionally further applying one or more secondary shortening elements thereto;
adhering said insulating layer to said backsheet, said insulating layer being sized and positioned to cover the at least one central track and the eventually at least one side tracks, to provide a laminated substrate;
providing an absorbent core comprising absorbent material;
providing a liquid permeable topsheet; and
sandwiching the absorbent core between said backsheet and said topsheet.

The method can further comprise the step of providing a slit and/or pocket on the backsheet enabling an electrically conductive portion of a clip-on data processing module to selectively come in electrical communication with the connection tracks of the conductive pattern.

All of these embodiments mentioned above can be taken individually or in combination.

Further embodiments are described below and in the claims.

### DESCRIPTION OF FIGURES

The drawings and figures are illustrative in nature and not intended to limit the subject matter defined by the claims. The following detailed description can be understood when read in conjunction with the following drawings, where like structure is indicated with like reference numerals in which:
Fig. 1 illustrates an isometric schematic view of an absorbent article according to an aspect of the present disclosure.
**Fig.** 2 represents a flow chart of the components of the sensor system according to the invention.
**Fig. 3a and 3b** each corresponds to a graph illustrating the evolution of a physical parameter of or within the absorbent article against time.
Fig. 4 illustrates a top view schematic of substrate according to an embodiment of the disclosure.
Fig. 5 illustrates a top view schematic of substrate according to an embodiment of the disclosure.
Fig. 6 illustrates a top view schematic of substrate according to an embodiment of the disclosure.
Fig. 7 illustrates a cross-sectional schematic of the substrate of Fig.6 about axis A-A.
Fig. 8 illustrates a top view schematic of substrate according to an embodiment of the disclosure.
**Fig.** 9 represents a flow chart of the components of the sensor system according to an embodiment of the invention.
**Fig.** 10 illustrates a top view schematic of substrate according to an embodiment of the disclosure.

### DETAILED DESCRIPTION OF THE INVENTION

The current invention concerns a sensor system configured for detecting a property of or within an absorbent article and an assembly of an absorbent article and said sensor system.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1 % or less, and still more preferably +/-0.1 % or less of and from the specified value, in so far such variations are appropriate to perform in the disclosed invention. However, it is to be understood that the value to which the modifier "about" refers is itself also specifically disclosed.

"Comprise," "comprising," and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The recitation of numerical ranges by endpoints includes all numbers and fractions subsumed within that range, as well as the recited endpoints except where otherwise explicitly stated by disclaimer and the like.

"Absorbent article" refers to devices that absorb and contain liquid, and more specifically, refers to devices that are placed against or in proximity to the body of the wearer to absorb and contain the various exudates discharged from the body. Absorbent articles include but are not limited to diapers, adult incontinence briefs, training pants, diaper holders and liners, sanitary napkins and the like, as well as surgical bandages and sponges. Absorbent articles preferably comprise a longitudinal axis and a transversal axis perpendicular to said longitudinal axis. The longitudinal axis is hereby conventionally chosen in the front-to-back direction of the article when referring to the article being worn, and the transversal axis is conventionally chosen in the left-to-right direction of the article when referring to the article being worn. Disposable absorbent articles can include a liquid pervious top sheet, a back sheet joined to the top sheet, and an absorbent core positioned and held between the top sheet and the back sheet. The top sheet is operatively permeable to the liquids that are intended to be held or stored by the absorbent article, and the back sheet may or may not be substantially impervious or otherwise operatively impermeable to the intended liquids. The absorbent article may also include other components, such as liquid wicking layers, liquid intake layers, liquid distribution layers, transfer layers, barrier layers, wrapping layers and the like, as well as combinations thereof. Disposable absorbent articles and the components thereof can operate to provide a body-facing surface and a garment-facing surface.

Preferably, a diaper comprises a liquid permeable "top sheet", a liquid impermeable "back sheet", and an "absorbent medium or core" disposed between the top sheet and the back sheet. The top sheet, back sheet and the absorbent medium could be made from any suitable material known to the person skilled in the art. The top sheet is generally located at or near the bodyside surface of the article, while the back sheet is generally located at or near the garment-side surface of the article. Optionally, the article may comprise one or more separate layers which are in addition to the back sheet and are interposed between the back sheet and the absorbent medium. Top sheet and back sheet are connected or otherwise associated together in an operable manner.

The "absorbent medium" or "absorbent core" or "absorbent body" is the absorbent structure disposed between the top sheet and the back sheet of the absorbent article in at least the crotch region of the absorbent article and is capable of absorbing and retaining liquid body exudates. The size and the absorbent capacity of the absorbent medium should be compatible with the size of the intended wearer and the liquid loading imparted by the intended use of the absorbent article. Further, the size and the absorbent capacity of the absorbent medium can be varied to accommodate wearers ranging from infants through adults. It may be manufactured in a wide variety of shapes (for example, rectangular, trapezoidal, T-shape, I-shape, hourglass shape, etc.) and from a wide variety of materials. Examples of commonly occurring absorbent materials are cellulosic fluff pulp, tissue layers, highly absorbent polymers (so called superabsorbent polymer particles (SAP)), absorbent foam materials, absorbent nonwoven materials or the like. It is common to combine cellulosic fluff pulp with superabsorbent polymers in an absorbent material.

"Acquisition and distribution layer", "ADL" or "surge management portion" refers to a sublayer which preferably is a nonwoven wicking layer under the top sheet of an absorbent product, which speeds up the transport and improves distribution of fluids throughout the absorbent core. The surge management portion is typically less hydrophilic than the retention portion, and has the ability to quickly collect and temporarily hold liquid surges, and to transport the liquid from its initial entrance point to other parts of the absorbent structure, particularly the retention portion. This configuration can help prevent the liquid from pooling and collecting on the portion of the absorbent garment positioned against the wearer's skin, thereby reducing the feeling of wetness by the wearer. Preferably, the surge management portion is positioned between the top sheet and the retention portion.

The term "adhesive" as used herein is intended to refer to any suitable hot melt, water or solvent borne adhesive that can be applied to a surface of a film layer in the required pattern or network of adhesive areas to form the film-nonwoven laminate of the present invention. Accordingly, suitable adhesives include conventional hot melt adhesives, pressure-sensitive adhesives and reactive adhesives (i.e., polyurethane).

As used herein, the term "adhesive bonding" means a bonding process which forms a bond by application of an adhesive. Such application of adhesive may be by various processes such as slot coating, spray coating and other topical applications. Further, such adhesive may be applied within a product component and then exposed to pressure such that contact of a second product component with the adhesive containing product component forms an adhesive bond between the two components.

As used herein, the term "associated or joined or adhered" encompasses, unless expressly stated, configurations in which e.g. a top sheet is directly joined to a back sheet by affixing the top sheet directly to the back sheet, and also configurations wherein the top sheet is joined to the back sheet by affixing the top sheet to intermediate members which in turn are affixed to the back sheet (i.e. indirectly joining). Top sheet and back sheet can be affixed directly to each other by attachment means such as an adhesive, sonic bonds, thermal bonds or any other attachment means known in the art. For example, a uniform continuous layer of adhesive, a patterned layer of adhesive, a sprayed pattern of adhesive or an array of separate lines, swirls or spots of construction adhesive may be used to affix top sheet to back sheet. It should be readily appreciated that the above-described attachment means may also be employed to interconnect and assemble together the various other component parts of the article described herein.

The terms "back section" and "rear back section" are used herein as synonyms and refer to the area of the absorbent article which is contact with the back of the wearer when the absorbent article is worn.

The term "backsheet" refers to a material forming a liquid impermeable cover of the absorbent article. The back sheet prevents the exudates contained in the absorbent structure from wetting articles such as bedsheets and overgarments which contact the disposable absorbent article. The back sheet may be a unitary layer of material or may be a composite layer composed of multiple components assembled side-by-side or laminated. The back sheet may be the same or different in different parts of the absorbent article. At least in the area of the absorbent medium the back sheet comprises a liquid impervious material in the form of a thin plastic film, e.g. a polyethylene or polypropylene film, a nonwoven material coated with a liquid impervious material, a hydrophobic nonwoven material, which resists liquid penetration, or a laminate of a plastic film and a nonwoven material. The back sheet material may be breathable so as to allow vapour to escape from the absorbent material, while still preventing liquids from passing there through. Examples of breathable back sheet materials are porous polymeric films, nonwoven laminates of spunbond and meltblown layers and laminates of porous polymeric films and nonwoven materials.

The terms "belly section" and "front belly section" are used herein as synonyms and refer to the area of the absorbent article which is contact with the belly of the wearer when the absorbent article is worn.

As used herein, the "body-facing" or "bodyside" surface means that surface of the article or component which is intended to be disposed toward or placed adjacent to the body of the wearer during ordinary use, while the "outward", "outward-facing" or "garment-side" surface is on the opposite side, and is intended to be disposed to face away from the wearer's body during ordinary use. Such outward surface may be arranged to face toward or placed adjacent to the wearer's undergarments when the absorbent article is worn.

"Bonded" refers to the joining, adhering, connecting, attaching, or the like, of at least two elements. Two elements will be considered to be bonded together when they are bonded directly to one another or indirectly to one another, such as when each is directly bonded to intermediate elements.

The term "breathable" refers to layers, preferably films or elastic laminates, having a water vapor transmission rate (WVTR) of at least 300 grams/m² - 24 hours.

The term "consisting essentially of" does not exclude the presence of additional materials which do not significantly affect the desired characteristics of a given composition or product. Exemplary materials of this sort would include, without limitation, pigments, antioxidants, stabilizers, surfactants, waxes, flow promoters, solvents, particulates and materials added to enhance processability of the composition.

Further, an absorbent article can comprise "containment flaps" or "barrier cuffs". The containment flaps are generally thought to be particularly well suited for the containment of fecal matter and to prevent the lateral flow of liquid waste until such time as the liquid waste can be absorbed by the absorbent article. Many constructions of containment flaps are known. Such containment flaps generally comprise a proximal edge, intended to be attached to the absorbent article, and an opposite distal edge which is generally not attached to the absorbent article along at least a portion of its length. An elastic member is generally located adjacent the distal edge to assist in maintaining the containment flap in an upright condition and in maintaining a sealing relationship between the distal edge of the containment flap and the body of a wearer during use. The elastic member is generally located between two layers of material so that the elastic does not come into contact with the body of a wearer. The containment flaps may be manufactured from a wide variety of materials such as polypropylene, polyester, rayon, nylon, foams, plastic films, formed films, and elastic foams. A number of manufacturing techniques may be used to manufacture the containment flaps. For example, the containment flaps may be woven, non-woven, spunbonded, carded, cast, blown or the like.

An absorbent article can comprise leg containment gaskets. Leg "containment gaskets" help prevent leakage of bodily exudates when the wearer exerts compressive forces on the absorbent article. In particular, the stiffness of the leg containment gaskets prevents twisting and bunching of the leg openings of the absorbent article which can lead to leaks. In addition, the elasticity and conformability of the leg containment gaskets ensures that the bodyfacing surface of the leg containment gaskets provides an adequate seal against the body of the wearer. The physical properties of the leg containment gaskets, such as the thickness and stiffness, also function to space the bodyside liner, outer cover and absorbent core away from the wearer's body when in use. As such, void volume is created between the wearer's body and the bodyside liner and absorbent core of the absorbent article to help contain body exudates.

"Mechanical bond" is an attachment between two or more elements, components, regions, or webs and may comprise heat bonds, pressure bonds, ultrasonic bonds, dynamic mechanical bonds, or any other suitable non-adhesive attachment means or combinations of these attachment means.

"Hotmelt adhesive" means a formulation that generally comprises several components. These components typically include one or more polymers to provide cohesive strength (e.g., aliphatic polyolefins such as poly (ethylene-co-propylene) copolymer; ethylene vinyl acetate copolymers; styrene-butadiene or styrene- isoprene block copolymers; etc.); a resin or analogous material (sometimes called a tackifier) to provide adhesive strength (e.g., hydrocarbons distilled from petroleum distillates; rosins and/or rosin esters; terpenes derived, for example, from wood or citrus, etc.); perhaps waxes, plasticizers or other materials to modify viscosity (i.e., flowability) (examples of such materials include, but are not limited to, mineral oil, polybutene, paraffin oils, ester oils, and the like); and/or other additives including, but not limited to, antioxidants or other stabilizers. For example, a typical hot-melt adhesive formulation might contain from about 15 to about 35 weight percent cohesive strength polymer or polymers; from about 50 to about 65 weight percent resin or other tackifier or tackifiers; from more than zero to about 30 weight percent plasticizer or other viscosity modifier; and optionally less than about 1 weight percent stabilizer or other additive. It should be understood that other adhesive formulations comprising different weight percentages of these components are possible.

"Discontinuous bonding pattern" as used herein refers to a pattern of bonding areas, in particular bonding areas between layers, whereby at least in at least one region the layers are not bonded. A discontinuous bonding pattern may comprise a connected bonding area or multiple disconnected bonding areas. A discontinuous bonding pattern further may comprise a connected bonding area comprising a number of holes, where the layers are not bonded, preferably according to a regular pattern, or it may comprise discrete disconnected bonding areas, e.g. a point bonded pattern which comprises a plurality of separate bonding points surrounded by unbonded areas or a line-bonded pattern which comprises a plurality of separate bonding lines alternated by unbonded areas, preferably according to a regular pattern.

The term "disposable" is used herein to describe absorbent articles that generally are not intended to be laundered or otherwise restored or reused as an absorbent article (i.e., they are intended to be discarded after a single use and, preferably, to be recycled, composted or otherwise disposed of in an environmentally compatible manner).

As used herein, the terms "elastic", "elastomeric", "elasticity" or derivations thereof are used to describe the ability of various materials and objects comprised of such to reversibly undergo deformation under stress, e.g., become stretched or extended, in at least one direction when a force is applied to the material and to resume substantially to their original dimensions upon relaxing, i.e., when the force is released, without rupture or breakage. Preferably, it refers to a material or composite which can be elongated in at least one direction by at least 50% of its relaxed length, i.e., elongated to at least 150% of its relaxed length, and which will recover upon release of the applied tension at least 40% of its elongation. Accordingly, upon release of the applied tension at 50% elongation, the material or composite contracts to a relaxed length of not more than 130% of its original length. Examples of suitable elastomer materials include polyether-polyamide block copolymers, polyurethanes, synthetic linear A-B-A and A-B block copolymers, chlorinated rubber/EVA (ethylene-vinyl acetate) blends, EPDM (ethylene-propylene diene monomer) rubbers, EPM (ethylene-propylene monomer) rubbers, blends of EPDM/EPM/EVA, and the like.

The term "elasticized" or "elastified" refers to a material, layer, or substrate that is naturally non-elastic, but which has been rendered elastic by, for example, suitably joining an elastic material, layer, or substrate thereto.

"Elongation" means the ratio of the extension of a material to the length of the material prior to the extension (expressed in percent). "Extension" means the change in length of a material due to stretching (expressed in units of length).

As used herein the term "extensible" means elongatable in at least one direction, but not necessarily recoverable.

The term "finished" or "final", when used with reference to a product, means that the product has been suitably manufactured for its intended purpose.

As used herein, the term "garment" means any type of apparel which may be worn. This includes diapers, training pants, incontinence products, surgical gowns, industrial workwear and coveralls, undergarments, pants, shirts, jackets and the like.

The term "graphic" includes, but is not limited to, any type of design, image, mark, figure, codes, words, patterns, or the like. For a product such as a training pant, graphics will generally include objects associated with little boys and little girls, such as multi-color trucks, airplanes, balls, dolls, bows, or the like.

As used herein, the term "impermeable" generally refers to articles and/or elements that are substantially not penetrated by aqueous fluid through the entire thickness thereof under a pressure of 1.0 kPa or less. Preferably, the impermeable article or element is not penetrated by aqueous fluid under pressures of 3.4 kPa or less. More preferably, the impermeable article or element is not penetrated by fluid under pressures of 6.8 kPa or less. An article or element that is not impermeable is permeable.

"Integral" is used to refer to various portions of a single unitary element rather than separate structures bonded to or placed with or placed near one another.

"Join", "joining", "joined", or variations thereof, when used in describing the relationship between two or more elements, means that the elements can be connected together in any suitable manner, such as by heat sealing, ultrasonic bonding, thermal bonding, by adhesives, stitching, or the like. Further, the elements can be joined directly together, or may have one or more elements interposed between them, all of which are connected together.

The term "laid-flat state" or "fully stretched state" or "extended state" is intended to refer to the article when it is flattened into a plane or is substantially flattened into a plane and is used in contrast to when the article otherwise positioned, such as when the article is folded or shaped in or for use by a wearer.

The use of the term "layer" can refer, but is not limited, to any type of substrate, such as a woven web, nonwoven web, films, laminates, composites, elastomeric materials, or the like, or even formulations, such as adhesives, that form a substrate upon a change in conditions (e.g. solidification of a hotmelt adhesive when the temperature drops below a predetermined amount). A layer can be liquid and air permeable, permeable to air but impermeable to liquids, impermeable both to air and liquid, or the like. When used in the singular, it can have the dual meaning of a single element or a plurality of elements.

The term "nonwoven fabric or web" means a sheet material having a structure of individual fibers or threads which are interlaid, but not in a regular manner such as occurs with knitting or weaving processes. Nonwoven fabrics or webs have been formed from many processes such as for example, meltblowing processes, spunbonding processes, and bonded carded web processes.

By the terms "particle", "particles", "particulate", "particulates" and the like, it is meant that the material is generally in the form of discrete units. The units can comprise granules, powders, spheres, pulverized materials or the like, as well as combinations thereof. The particles can have any desired shape such as, for example, cubic, rod-like, polyhedral, spherical or semi-spherical, rounded or semi-rounded, angular, irregular, etc. Shapes having a large greatest dimension/smallest dimension ratio, like needles, flakes and fibers, are also contemplated for inclusion herein. The terms "particle" or "particulate" may also include an agglomeration comprising more than one individual particle, particulate or the like. Additionally, a particle, particulate or any desired agglomeration thereof may be composed of more than one type of material.

Use of the term "substrate" includes, but is not limited to, woven or nonwoven webs, porous films, ink permeable films, paper, composite structures, or the like.

Superabsorbent materials (e.g. superabsorbent polymers) suitable for use in the present disclosure are known to those skilled in the art, and may be in any operative form, such as particulate form, fibers and mixtures thereof. Generally stated, the "superabsorbent material" can be a water-swellable, generally water-insoluble, hydrogel-forming polymeric absorbent material, which is capable of absorbing at least about 15, suitably about 30, and possibly about 60 times or more its weight in physiological saline (e.g. saline with 0.9 wt % NaCl). The superabsorbent material may be biodegradable or bipolar. The hydrogel-forming polymeric absorbent material may be formed from organic hydrogel-forming polymeric material, which may include natural material such as agar, pectin, and guar gum; modified natural materials such as carboxymethyl cellulose, carboxyethyl cellulose, and hydroxypropyl cellulose; and synthetic hydrogel-forming polymers. Synthetic hydrogel-forming polymers include, for example, alkali metal salts of polyacrylic acid, polyacrylamides, polyvinyl alcohol, ethylene maleic anhydride copolymers, polyvinyl ethers, polyvinyl morpholinone, polymers and copolymers of vinyl sulfonic acid, polyacrylates, polyacrylamides, polyvinyl pyridine, and the like. Other suitable hydrogel-forming polymers include hydrolyzed acrylonitrile grafted starch, acrylic acid grafted starch, and isobutylene maleic anhydride copolymers and mixtures thereof. The hydrogel-forming polymers may be lightly crosslinked to render the material substantially water insoluble. Crosslinking may, for example, be by irradiation or covalent, ionic, Van der Waals, or hydrogen bonding. The superabsorbent material may suitably be included in an appointed storage or retention portion of the absorbent system, and may optionally be employed in other components or portions of the absorbent article. The superabsorbent material may be included in the absorbent layer or other fluid storage layer of the absorbent article of the present invention in an amount up to about 60% by weight.

Typically, the superabsorbent material, when present, will be included in an amount of about 5% to about 40% by weight, based on the total weight of the absorbent layer.

"Superabsorbent polymer particles" or "SAPs" refer to water-swellable, water-insoluble organic or inorganic materials capable, under the most favorable conditions, of absorbing at least about 10 times their weight, or at least about 15 times their weight, or at least about 25 times their weight in an aqueous solution containing 0.9 weight percent sodium chloride. In absorbent articles, such as diapers, incontinent diapers, etc., the particle size is typically ranging between 100 to 800 µm, preferably between 300 to 600 µm, more preferably between 400 to 500 µm.

The term "topsheet" refers to a liquid permeable material sheet forming the inner cover of the absorbent article and which in use is placed in direct contact with the skin of the wearer. The top sheet is typically employed to help isolate the wearer's skin from liquids held in the absorbent structure. The top sheet can comprise a nonwoven material, e.g. spunbond, meltblown, carded, hydroentangled, wetlaid etc. Suitable nonwoven materials can be composed of man-made fibres, such as polyester, polyethylene, polypropylene, viscose, rayon etc. or natural fibers, such as wood pulp or cotton fibres, or from a mixture of natural and man-made fibres. The top sheet material may further be composed of two fibres, which may be bonded to each other in a bonding pattern. Further examples of top sheet materials are porous foams, apertured plastic films, laminates of nonwoven materials and apertured plastic films etc. The materials suited as top sheet materials should be soft and non-irritating to the skin and be readily penetrated by body fluid, e.g. urine or menstrual fluid. The inner coversheet may further be different in different parts of the absorbent article. The top sheet fabrics may be composed of a substantially hydrophobic material, and the hydrophobic material may optionally be treated with a surfactant or otherwise processed to impart a desired level of wettability and hydrophilicity.

As used herein, the term "transverse" or "lateral" refers to a line, axis, or direction which lies within the plane of the absorbent article and is generally perpendicular to the longitudinal direction.

"Ultrasonic welding" refers to a technology which joins two materials by melting them with heat generated from ultrasonic oscillation and then laminating them together, such that the molten materials flow and fill the gap between the two unaffected portions of the two materials, respectively. Upon cooling and shaping, the two materials are joined together.

"Form-driven locking" refers to locking via the shape of an element, such as a key lock wherein the shape of an element provides a resisting force to the opening thereof.

"Force-driven locking" refers to locking via a means selected from the group consisting of electromechanical, magnetic, adhesive and combinations thereof.

"Substantial portion" as used herein with reference to a element/component (e.g. wetness sensing tracks), refers to at least 80%, preferably at least 90%, more preferably at least 95%, of said element/component, generally measured as a surface area taken in the laid flat state of the absorbent article.

"Substantially perpendicular (or parallel)" refers to an element extending at an angle of not more than 35°, preferably less than 25°, more preferably less than 20°, even more preferably less than 15°, even more preferably less than 10°, most preferably less than 5°, from the referred perpendicular (or parallel) axis.

Embodiments according to the disclosure will now be described. It is understood that technical features described in one or more embodiments maybe combined with one or more other embodiments without departing from the intention of the disclosure and without generalization therefrom.

### ABSORBENT ARTICLE

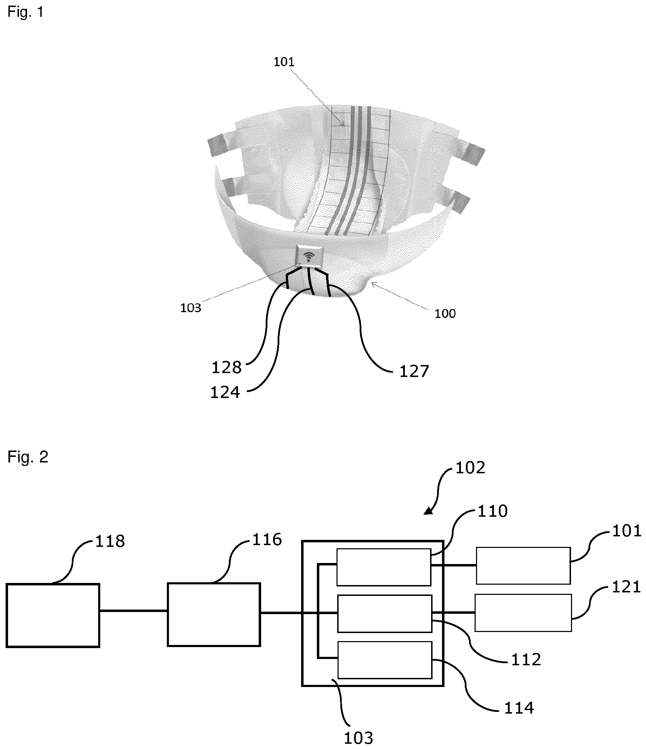

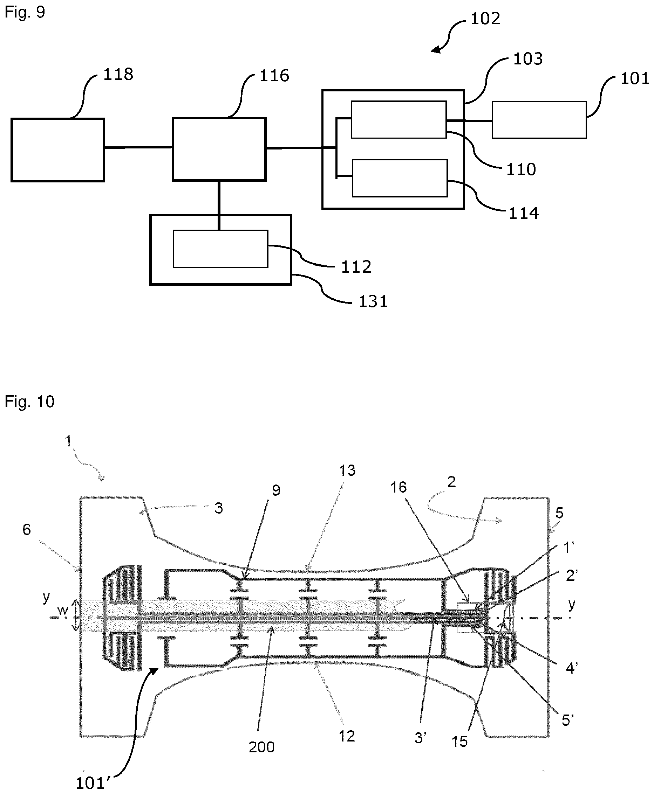

In an embodiment, the absorbent article 100 according to the present disclosure as exemplified in Fig. 1, is typically a disposable diaper or pant, and suitable for detecting a wetness or spill (i.e. urine, feces and/or other bodily fluids) event therein and/or risk of exudate leakage therefrom, said absorbent article 100 comprising: a liquid impermeable backsheet; a liquid permeable topsheet; and an absorbent core interposed between said backsheet and topsheet, wherein said backsheet comprises a substrate 101 that will be described hereafter, typically the backsheet, absorbent core and topsheet forming a chassis of the absorbent article 100. The absorbent article 100 comprises a sensor system 102, illustrated in Fig. 2 and 9, configured for detecting a property of or within said absorbent article 100. The absorbent article 100, specifically the substrate 1, has a first end 5 (and/or backsheet) corresponding to the front (or belly portion) of the absorbent article 100 and a second end 6 corresponding to the back of the absorbent article 100 with lateral side edge 12,13.

The absorbent article may further comprise additional components that are common in the art and selected from the group consisting of: a liquid distribution layer (ADL) positioned between the topsheet and the absorbent core; elastic or non-elastic back ears joined to the chassis at a position proximal to the second end of the substrate 1 forming the backsheet; super absorbent polymer particles and/or fibers typically comprised within the absorbent core; cellulose fibers typically comprised within the absorbent core; fastening tapes joined to the back ears for adhesive and/or mechanical coupling with a garment facing surface of the backsheet at a position proximal to the first end; elastic waistband positioned proximal to the first and/or second ends; and combinations thereof. When the absorbent article is a pant, the absorbent article may comprise a front belt positioned proximal to the first end and a rear belt positioned proximal to the second end, said belts being separated from each other by the chassis of the absorbent article and being directly joined to each other via two opposite side seams, said belts typically being elastic.

In an embodiment, the absorbent core comprises a nonwoven core wrap typically arranged such that there is no folding (i.e. no nonwoven overlap) of said core wrap coming in direct contact with the backsheet comprising the sensor tracks.

### SENSOR SYSTEM

The sensor system 102 according to the present invention and as illustrated in Fig. 2 and 9, comprises a first sensor 110 configured to measure a first physical parameter, said first sensor 110 being configured to detect a change in condition of or within the absorbent article 100 and a second sensor 112 configured to measure a second physical parameter, said second sensor 112 being configured to sense a change in condition of or within the absorbent article 100. According to the invention, the first and second physical parameters are different from one another.

By "physical parameter" it is implied a measurable physical characteristic of or within the absorbent article, including, but not limited to, temperature, electrical conductance or electrical resistance, electrical capacitance, pH, mass, solubility, pressure, length, volumetric flow. Therefore the first sensor 110 measures a first physical characteristic, for example and not limited to, temperature, while the second sensor 112 measures a different physical characteristic, for example and not limited to, electrical resistance. It is therefore possible to better assess the condition of or within the absorbent article 100 and determine if it needs to be changed or not.

The sensor system 102 according to the invention enables to measure two different physical parameters of or within the absorbent article. A physical parameter has its own evolution throughout time, thereby by monitoring two distinct physical parameters that each evolve differently throughout time, it is possible to have a better reading of the condition of or within the absorbent article 100 and assess if its need to be changed or not.

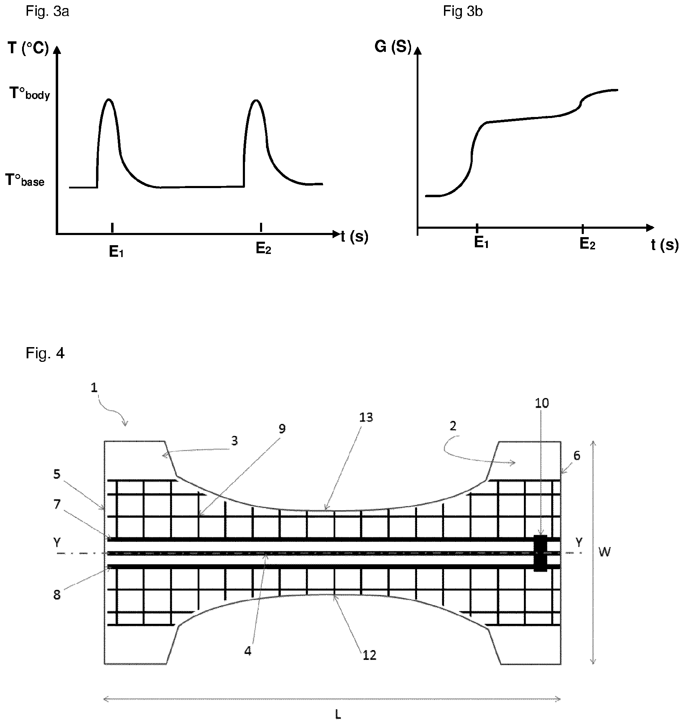

Fig. 3a and Fig. 3b represents two graphs showing the evolution of a physical parameter of or within an absorbent article throughout time. Fig. 3a illustrates the evolution of Temperature (T° in degree Celsius °C) (Ordinate) of or within the absorbent article 100 against time (t in second s)(abscissa). Fig. 3b illustrates the evolution of conductance (G in Siemens S) (Ordinate) of or within the absorbent article 100 against time (t in second s)(abscissa). Electrical resistance is the reciprocal quantity of electrical conductance (G=1/R), so when measuring one of these parameters it is easy to obtain the other.

Temperature is a parameter adapted for detecting the precise moment of a wetness/spill/exudate event, as said event causes a spike in temperature. Indeed, the usual temperature of or within an absorbent article is base temperature T°_{base} which is comprised between room temperature (around 20°C) up to the skin temperature (around 34°C, depending on the individual person wearing the absorbent article). During a wetness/spill/exudate event (E₁), bodily fluids are expulsed within the absorbent article 100, said fluids being at the body temperature T°_{body} (around 37°C, depending on the individual) thus causing a rise in temperature. This temperature spike can easily be detected by a temperature sensor. Once the first exudate wetness/event (E₁) has passed, the bodily fluids will cool down and the temperature of or within the absorbent article cools down to base temperature T°_{base}. A second wetness/exudate event (E₂) will provoke another spike in temperature. Thus by monitoring the evolution of temperature throughout time, it is possible to see how many wetness/exudate events (Eₙ) a person has undergone through. However, this parameter is qualitative and gives little information as to the amount of bodily fluids that has been expulsed.

Electrical conductance, or electrical resistance, is a parameter adapted for detecting the amount of bodily fluids expulsed during a wetness/spill/exudate event (Eₙ), as said event causes an increase in electrical conductance (or a drop in electrical resistance). The amount of bodily fluids alters the level of electrical conductance of or within the absorbent article. More specifically, bodily fluids increase the electrical conductance, or decrease electrical resistance, by releasing mineral salts (Na⁺, Cl⁻, NH₄⁺, K⁺) which enable a good electrical conductance, or poor electrical resistance, of or within the absorbent article 100. Thus a wetness/spill/exudate event (Eₙ) corresponds to an increase in electrical conductance. By measuring the level of electrical conductance, or electrical resistance, precisely (which correspond to the concentration of mineral salts), it is possible to determine the amount, or level, of bodily fluids expulsed. However after a first wetness/spill/exudate event (E₁), the level of electrical conductance does not lower, *i.e.* the level of electrical conductance does not revert to a base value. A second wetness/spill/exudate event (E₂) further increases the concentration in mineral salts and the electrical conductance of or within the absorbent article 100 further increases (or electrical resistance will further drop). However this second increase in electrical conductance represents a small increase compared to increase in electrical conductance caused by the first wetness/exudate event (E₁). Thus, by monitoring the evolution of electrical conductance, or electrical resistance, throughout time, it is possible to see the amount of bodily fluids that has been expulsed but it is harder to define the number of wetness/exudate event. The same reasoning can be applied to electrical resistance, where a wetness/exudate event will diminish the electrical resistance of or within the absorbent article.

The apparatus to measure electrical resistance, or electrical conductance, and temperature are described in the FIRST SENSOR section and SECOND SENSOR section respectively. Combining the monitoring of both parameters is particularly beneficial as it enables to have a quantitative and qualitative monitoring of the condition of or within the absorbent article 100 throughout time. A care giver can determine if a person underwent too many wetness, spill, soilage and/or exudate events and if the absorbent article 100 has absorbed a targeted amount of bodily fluids in order to change the absorbent article at the optimal time to avoid discomfort of the patient or baby. According to one embodiment, the sensor system monitors the temperature and conductance (T°,G) behaviour of or within an absorbent article throughout time by measuring temperature and conductance (T°,G) values and plotting said values against time. According to a further embodiment, the sensor system monitors the temperature, conductance and positioning (T°, G, (x,y,z)) behaviour of or within an absorbent article throughout time by measuring temperature, conductance and positioning (T°, G, (x,y,z)) values and plotting said values against time.

The sensor system 102 according to the invention is not limited to these two physical parameters exclusively. Other physical parameters can be monitored in order to have a quantitative and qualitative monitoring of the condition of or within the absorbent article 100 throughout time. For example, and not limited to, it is possible to monitor temperature and capacitance or temperature and absorption level (amount of water absorbed).

The sensor system 102 according to the invention is not limited to the monitoring of two different physical parameters exclusively. The sensor system 102 may monitor a third physical parameter. According to an embodiment, the sensor system comprises a third sensor 114 to monitor a third physical parameter different from the two physical parameters measured by the first sensor 110 and the second sensor 112. The third sensor 114 is for example, but not limited to, a motion sensor in order to determine the position of the absorbent article within a space, i.e. a three-dimensional extent, such as a room or an area including and around a bed for example.

The sensor system 102 according to the invention is no limited to the monitoring of two or three different physical parameters exclusively, it is possible to monitor four or more different physical parameters.

The second sensor 112 may be distinct and remote from the first sensor 110. The third sensor 114 may be distinct and remote from the second sensor 112 as well as from the first sensor 110. By being distinct and remote, it is possible to have different power sources for each sensor 110,112,114 and have a safe-fail system for the sensor system 102 in the sense that it is still possible to detect a property of or within an absorbent article 100 should one sensor malfunction.

As explained above, the first sensor 110 is configured to measure electrical resistance, or electrical conductance, and the second sensor 112 is configured to measure temperature. The third sensor 114 is configured to measure motion, i.e. the positioning of the absorbent article 100 within a space.

Sensors configured to measure electrical conductance usually have high power consumption. In a sensor system 102 according to an embodiment, the first sensor 110 is activated directly or indirectly by the second sensor 112.

As illustrated in Fig. 2, the sensor system 102 according to the invention may further comprise a control system 116 that manages the data outputs of first and second sensors 110,112, namely electric resistance, or electrical conductance, and temperature values, and emits output data to an user interface 118. The control system 116 can include a processor and memory. The processor can perform various computing functions, such as those described before, *i.e.* retrieving temperature, electrical resistance, electrical conductance and/or positioning values and plotting said values against time measured by an internal clock that can be synchronized with the clock of the user interface 118. The processor can perform other computing functions useful for operating the sensors 110, 112, 114. The memory can be a non-transient computer readable storage medium (e.g., random access memory or flash) for storing program instructions and/or frames. For example, the processor can be configured to execute program instructions stored on the memory for controlling the sensors into digital signals, storing the digital signals on the memory as frame data, transferring the frame data to the computing system, transferring the results of the computing system to the user interface 118 and/or performing any other function. The processor may perform various signal conditioning and/or image processing. The processor may include a dedicated video processor for image processing.

The control system 116 can comprise a single housing or multiple housings among which circuitry can be distributed. The control system 116 and its computing system can include display circuitry which can provide a graphics output to a screen, namely the user interface 118. Display circuitry can include a graphics processor and graphics memory which can support user interface functionality. Display circuitry may be part of a separate display, such as a screen, handheld device, or remote terminal. Display circuitry can facilitate the display of frames taken by the camera of the patient area on a screen and/or patient status information. User input circuitry can include components for accepting user commands such as a keyboard, mouse, trackball, touchpad, touch screen, joystick, slider bar, or any other control. User input circuitry can facilitate the definition of boundaries and monitoring zones such as a zone occupied by one or a plurality of patients.

As explained above, the computing system of the control system 116 can include a processor and memory. The memory can be one or more discrete non-transient computer readable storage medium components (e.g., RAM, ROM, NYRAM, EEPROM, and/or FLASH memory) for storing program instructions and/or data. The processor can be configured to execute program instructions stored on the memory to control in the computing system of the control system 116 in carrying out the functions referenced herein. The processor can comprise multiple discrete processing components to carry out the functions described herein as the processor is not limited to a single processing component. The computing system can include a network controller for facilitating communication with the sensors and/or other remote components.

The control system 116 can include a power supply which can facilitate a connection to an electrical outlet and/or the power supply can comprise a battery. Whether distributed or unified, the components of the computing system of the control system 116 can be electrically connected to coordinate and share resources to carry out functions.

### FIRST SENSOR

The sensor system 102 according to the invention comprises a first sensor 110. The first sensor 110 may be configured to measure electrical resistance, or electrical conductance, of or within the absorbent article 100.

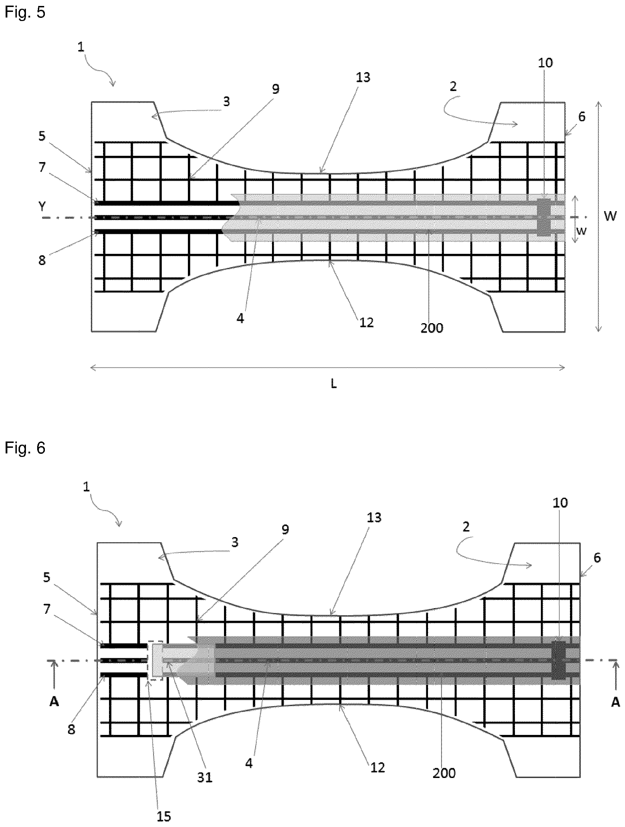

In an embodiment, as shown in exemplary Fig. 4 to 8, the substrate 1, suitable for incorporation into an absorbent article 100 for automatic detection of wetness (and/or exudate) events therein, comprises a first surface 2 capable of being arranged proximal to a body facing side of the absorbent article 100 and a second surface 3 opposite said first surface 2 and capable of being arranged proximal to a garment facing side of said absorbent article 100, said substrate 1 comprising a plurality of sensor tracks 101 disposed on said first surface 2 and said sensor tracks 101 comprising: at least one central track 4 extending parallel to a length L of the substrate and parallel to a longitudinal axis y-y crossing a first end 5 and a second end 6 of the substrate 1; at least one or more, here two, side tracks side tracks 7,8 extending parallel to the central track 4 and oppositely arranged such that the central track 4 extends therebetween; and wetness sensing tracks 9 extending outboard of said at least one or more side tracks 7,8, wherein said central track 4, said at least one or more side tracks 7,8, and said wetness sensing tracks 9 are in electrical communication via one or more shortening elements 10 positioned proximal to said second end 6 and distal from said first end 5. The substrate 1 is connectable to a clip-on data processing module 103 at a position proximal to said first end 5 and distal from said shortening elements 10 such to form a closed electrical circuit, typically for measuring electrical resistance, *i.e.* electrical conductance, and/or capacitance therethrough.

It has been advantageously found that a substrate that comprises the above described sensor track arrangement provides for accurate detection of wetness/spill/soil/exudates as well as being particularly suitable for fully scalable in-line mass production at high speeds, without the need to change arrangement depending on product sizes. For example, an advantage of positioning the wetness sensing tracks outboard of the other tracks is that if/when the substrate is cut to form for example leg openings (which may vary depending on product size), the circuitry is not damaged (since e.g. the central feedback track would always remain intact) and wetness detection is still ensured across the areas of greater risk of leakage that are located proximal to the edges and/or sides of the absorbent article.

In an embodiment, the distance between the at least two side tracks 7,8 is less than 0.3W, wherein W is the width of the substrate 1 extending perpendicular to the length L thereof. The distance between each side track 7,8 and lateral side edges 12,13 of the substrate 1 is greater than 0.2W, preferably greater than 0.25W, even more preferably from greater than 0.26W to less than 0.5W.

In a preferred embodiment, the substrate 1 consists of a liquid impermeable backsheet, preferably a breathable liquid impermeable backsheet.

In an embodiment, the wetness sensing tracks 9 are characterized by resistance (or more generally speaking impedance) thus conductance values, again resistance being the reciprocal value of conductance (R=1/G). Indeed, resistive, or conductive, measurements described herein are not limited to direct current (DC) power sources but also and most preferably alternating current (AC) power sources. Said wetness sensing tracks 9 generally having an adjusted resistance/impedance design, that can further be defined by a mathematical distribution model in the variation of respective resistance/impedance values over a surface of the absorbent article, said distribution model may be a linear, quadratic or logarithmic, preferably logarithmic, distribution.

In a preferred embodiment, the resistance/impedance of the wetness sensing tracks 9 proximal to the lateral side edges 12,13 and/or first/second ends 5,6 of the substrate 1 is greater than on any other portion of said substrate 1 (typically said other portions being inboard of said lateral side edges 12,13 and/or first/second ends 5,6 and proximal to the middle and/or center of the substrate 1). In an alternative embodiment said resistance/impedance is substantially the same throughout the wetness sensing tracks 9.

Especially, but not only, in the latter embodiment (i.e. also independently therefrom) the wetness sensing tracks 9 may form a grid pattern across the substrate 1 wherein said grid comprises resistive (and/or conductive) elements running both substantially parallel to the longitudinal axis y-y as well as substantially perpendicular thereto. The latter arrangement is beneficial for ensuring reliable sensing of wetness events also upon saturation of the product which inevitably results in expansion of the absorbent core of the absorbent article and thus more prone to delamination of the absorbent core components (e.g. the core wrap) from the backsheet. Surprisingly such a grid stucture has been found to be beneficial in providing continued and reliable detection also in such extreme conditions.

Preferably, the sensor tracks 101 comprise an electrically conductive material, and are preferably printed sensor tracks. In a preferred embodiment, the printed sensor tracks 101 comprise, preferably consist of, a carbon-based ink and/or a conductive polymer-based ink, preferably the carbon-based ink comprising a conductive compound selected from the group consisting of graphene, graphite, nano-carbon-tubes and mixtures thereof, preferably the conductive polymer-based ink comprising a conductive compound selected from the group consisting of polyacetylene, polypyrrole, polyaniline and copolymers thereof, more preferably selected from the group consisting of poly(pyrrole)s (PPY), polyanilines (PANI), poly(thiophene)s (PT), poly(p-phenylene sulfide) (PPS), poly(p-phenylene) (PPP), Poly(acetylene)s (PAC), Poly(p-phenylene vinylene) (PPV), poly(3,4-ethylenedioxythiophene) (PEDOT), and mixtures thereof, most preferred conductive polymer-based ink comprising poly(3,4-ethylenedioxythiophene) polystyrene sulfonate (PEDOT:PSS). An advantage of this arrangement is that fast production is achieved whilst ensuring good compatibility with the end/final product unlike with the addition of foreign conductive elements like copper wires and the like, which are more prone to tearing the liquid impermeable substrate.

In a highly preferred embodiment, the substrate 1 further comprising an insulating layer 200, preferably being liquid impermeable, adhered thereto and sized to cover the at least one central track 4 and the side tracks 7,8, said insulating layer 200 adapted to provide a seal and/or barrier to liquid from coming into contact with said central track 4 and said side tracks 7,8, preferably wherein the wetness sensing tracks 9 remain exposed and are not covered by said insulating layer 200. An advantage of this arrangement is that the insulating layer protects the central tracks and side tracks from coming into direct contact with exudates and thus limiting the risk of false positives, noise and even failure of detection.

Preferably, the insulating layer 200 comprises the one or more shortening elements 10, such that the central track 4, the side tracks 7,8, and the wetness sensing tracks 9 are in electrical communication via said shortening elements 10 when said insulating layer 200 is joined to the substrate 1. An advantage of this arrangement is that it allows a non-registered process to be used in processing the substrate, indeed registered printing of backsheets would add complexity and cost to the overall production.

In an embodiment, the substrate 1 comprises at least two, preferably at least three, central tracks 4 extending parallel to each other, and further being in electrical communication with each other via one or more secondary shortening elements 11, preferably said secondary shortening elements 11 being located distal from the shortening elements 10 and between said shortening elements 10 and the first end 5 of the substrate 1 typically such to provide one or more resistance identifiers, most preferably wherein the insulating layer 200 comprises said secondary shortening elements 11. An advantage is that identifiers such as the level of absorbency of the absorbent article, the size etc. can be pre-defined and detected when connected to the clip-on module, based on the location of the secondary shorts generating a pre-set resistance level that the module can detect by comparing for example the initial resistive measurement upon connection to the absorbent article versus preset values that may be stored in a memory within the unit.

When the substrate 1 comprises two or more central tracks 4, the longitudinal axis y-y may extend in between at least two of said two or more central tracks 4. An advantage of this arrangement is that it enables reliable location of the clip on module for correct and appropriate electrical connection to the respective tracks.

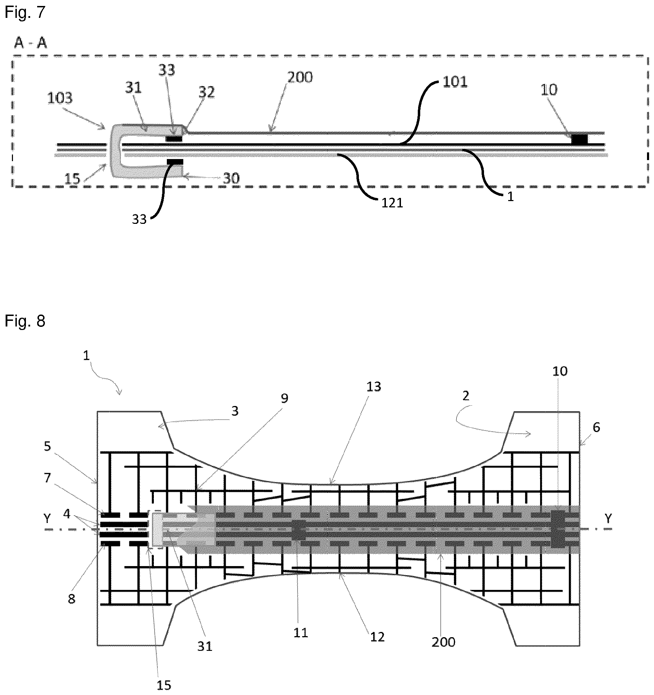

In an embodiment as shown in exemplary Fig. 8, at least one, preferably each, of the side tracks 7,8 is discontinuous along an axis parallel to the length L of the substrate 1 such to force a current through the wetness sensing tracks 9 towards regions proximal to lateral edges 12,13 of the substrate 1 said lateral edges 12,13 extending between the first and second ends 5,6 and along the length L of the substrate 1, preferably wherein at least a portion of the wetness sensing tracks 9 are further alternatingly directly connected and disconnected to the respective side tracks 7,8 along the length L of the substrate 1. Preferably said portion of the wetness sensing tracks 9 is proximal to the first and/or second ends 5,6 of the substrate 1. An advantage of this arrangement is that better accuracy of exudate detection is achieved where it is needed most for assessing risk of leakage i.e. proximal to the sides and/or ends of the substrate. Moreover, by having an alternating connection of the wetness sensing tracks as shown in Fig.8 in regions proximal to the first and/or second ends, improved detection capability is enhanced also proximal to the central portion of the substrate in regions where further potential leakage and/or discomfort may occur.

In an embodiment, the wetness sending tracks 9 are non-evenly distributed across the surface of the substrate 1 and arranged such that portions of said wetness sensing tracks proximal to the lateral edges 12,13 of the substrate 1 are thinner than those portions distal therefrom and proximal to the center of the substrate. Preferably, tracks running perpendicular to the longitudinal axis y-y are thinner than those running parallel thereto.

The wetness sending tracks 9 are in communication with the first sensor 110 located in the clip-on module 103 that is described hereunder in the appropriate section.

In an embodiment, the absorbent article comprises a removable clip-on data processing module 103, that is further described hereunder in the appropriate section, adapted to monitor and process resistance and/or capacitance data acquired from the wetness sensing tracks 9, said clip-on data processing module 103, and in particular the first sensor contained in the clip-on module 103, being connectable to the at least one central track 4 and the at least one side track 7,8 via a slit 15 and/or pocket on the backsheet enabling an electrically conductive portion of said module 103 to directly come in electrical communication with said at least one central track 4 and said at least two side tracks 7,8, preferably the slit and/or pocket arranged to accommodate at least 50%, preferably at least 60%, more preferably at least 70%, even more preferably at least 80%, of the total surface area of the clip-on data processing module 103 therein. An advantage of this arrangement is that the module remains nicely secured to the absorbent article as well as being protected from accidental disengagement during movements of the subject/wearer.

In an embodiment, the insulating layer 200 comprises the one or more shortening elements 10, such that the central track 4, the side tracks 7,8, and the wetness sensing tracks 9 are in electrical communication via said shortening elements 10 only when said insulating layer 200 is joined to the substrate 1. An advantage of this arrangement is that a greater degree of flexibility is provided as well as simplifying the process of assembly for reasons that will be better explained in the process section herein.

In an embodiment, the backsheet comprises a slit 15 at a location proximal to the first end 5 of the substrate where the clip-on data processing module 103, in particular the first sensor 110, is to be connected, said slit being sized such to receive therethrough, typically only, a flexible connection member 31 typically comprising connection ports 33 of the clip-on module 103 and wherein, once said clip-on data processing module 103, in particular the first sensor 110, is connected, said backsheet is interposed between the free ends 32 of the flexible connection member 31 and the housing 30 of the clip-on module 103 such that said free ends 32 does not come in direct contact with said housing at a position proximal to said free end 32, preferably wherein the housing 30 is positioned on a garment facing side of the backsheet and the free end 32 of the flexible connection member 31 on a body facing side of the backsheet. An advantage of this arrangement is that a secure connection may be achieved between the module 103 and the chassis of the article and in particular a very secure electrical connection to the sensor tracks is enabled, whilst it being protected from soiling by internal and/or external elements like dust, exudates, food and the like.

Another embodiment relating to the arrangement of the sensor tracks 101 is illustrated in Fig. 10. It is understood that elements of the sensor system and absorbent article described previously can be identical or similar and have the same function and that reference numerals may refer to similar or identical elements with no limitation or restriction to the present invention. In this embodiment, the substrate 1 suitable for incorporation into the absorbent article 100 for monitoring and detecting the presence of wetness therein and/or risk of wetness, spill, soilage or exudate leakage therefrom, is provided. Said substrate 1 comprises a conductive pattern 101', corresponding to the sensor tracks 101 as described above, disposed on the first surface 2 capable of being arranged proximal to a body facing side of the absorbent article 100, wherein said conductive pattern can be brought in electrical communication with the clip-on data processing module 103. Said conductive pattern 101' comprises: a plurality of connection tracks, exemplary connection tracks 1',2',3',4',5'; and a plurality of wetness sensing tracks 9 connected to said connection tracks 1',2',3',4',5', and wherein the conductive pattern 101' is configured in a manner that addressing multiple combinations of said plurality of connection tracks, for example tracks 1',2',3',4',5', with corresponding terminals of the clip-on data processing module 103, for example the terminals 33 correspond to the connection ports 33 described previously, results in multiple electrical circuit configurations for measuring resistance, conductance, impedance and/or capacitance therethrough.

Although here, five connection tracks 1',2',3',4',5' are shown, a different number of connection tracks can be used. Especially, 3, 4, 5, 6, 7, 8, 9, 10, 11, 12, 13, 14, 15, 16, 17, 18, more than 3, more than 4, more than 5, more than 6, more than 7, more than 8, more than 9, more than 10, more than 11, more than 12, more than 13, more than 14, more than 15, more than 16, more than 17, more than 18 connection tracks may be used. The higher the number of connection tracks, the more permutations of connection tracks are possible, resulting in a greater number of distinguishable sensing zones, and therefore in a finer localization of the intake.

In the context of Fig. 10, the substrate 1 further comprises a second surface 3 opposite said first surface 2 and capable of being arranged proximal to a garment facing side of said absorbent article 100. The clip-on data processing module 103 can be in electrical communication with the conductive pattern 101' when connected at a position proximal to a first end 5 of the substrate 1, such to form at least one closed electrical circuit.

The connection tracks, for example tracks 1',2',3',4',5', are configured to allow an electrical current to flow from corresponding terminals 33, or connection ports 33, of the clip-on data processing module 103, exemplary connection terminals when connected thereto. Additionally or alternatively, said connection tracks may also be configured to allow an electrical current to flow to, or from, corresponding terminals 33, or connection ports 33, of the clip-on data processing module 103.

It may be beneficial that said connection tracks, exemplary tracks 1',2',3',4',5', extend substantially parallel to a length of the substrate and substantially parallel to a longitudinal axis (y-y) crossing a first end 5 and a second end 6 of the substrate 1. Moreover, the sensing tracks 9 are configured to undergo changes in presence of certain substances, such as stool and/or cellulose fibers combined with superabsorbent polymers saturated with urine. Advantageously, it has been found that a substrate comprising the above described conductive pattern 101' provides for accurate detection of exudates as well as being particularly suitable for fully scalable in-line mass production at high speeds, without the need to change arrangement depending on product sizes.

Further advantageously, the conductive pattern 101' mentioned above allows for multiple usage of terminals, i.e. the connection ports 33, of the clip-on data processing module 103, which in turn enables to detect wetness along different, specific zones of the absorbent article 100. Preferably, the conductive pattern 101' comprises an electrically conductive material, as described previously and is preferably a printed conductive pattern, so that the distribution of said conductive pattern 101' is registered and, hence, the sensor design is directly comprised on the substrate 1. This is advantageous, since further modifications as e.g. in order to characterize the absorbent product 100 (including for example information regarding the product size, absorbency of the core or the like), can be directly comprised within the printed conductive pattern. In addition to this, said sensor design may also comprise registration marks, arranged to provide a trigger to a registering device to carry out process steps.

In the embodiment as depicted in Fig. 10, the substrate 1 can further comprises at least one insulating layer 200, as described previously, preferably being liquid impermeable, adhered thereto and sized to cover at least a portion of the conductive pattern 101' (typically said "at least portion" being proximal to a position where the clip-on data processing module 103 is connected to said substrate). Said at least one insulating layer 200 is adapted to provide a seal and/or barrier to liquid and/or exudates from coming into contact with said portion of the conductive pattern 101', preferably wherein the sensing tracks 9 remain exposed to liquids and/or exudates and are not covered by said at least one insulating layer 200. Advantageously, the at least one insulating layer protects portions of the conductive pattern 101' to come into direct contact with exudates, limiting the risk of false positives, noise and even failure of detection.

As depicted in Fig. 10, the substrate 1 comprises one or more slits 15 for insertion of the clip-on module 103. In addition to this, a pocket 16 is formed between the first surface 2 and the at least one insulating layer 200 proximal to the first end 5, said pocket 16 being in fluid communication only with said slit(s) 15 and arranged to retain at least a portion of the clip-on data processing module 103 therein. This is beneficial, since an effective retaining pocket is attained in a cheap and scalable manufacturing process allowing for manufacturing of such substrates at high production speeds which still ensures a pocket is formed to retain the clip-on module 103 in contact with the connection tracks whilst at the same time providing insulation against exudates as the substrate becomes saturated. Preferably, the pocket 16 is sized such that it matches the dimensions of a pocket insertion member of the clip- module 103 that typically comprises terminals 33 or connection ports 33, such that said terminals 33 or connection ports 33 come into electrical communication with respective connection tracks 1',2',3',4',5' by simply inserting the clip-on data processing module 103 into the pocket 16 until a pocket dimension stops it from being inserted any further. Advantageously, this allows the caregiver to connect said clip-on module 103 without having to worry about particularly positioning the module to ensure good electrical connection.

For insertion of the module, an opening cut (or slit) can be created within the substrate. The opening cut can be a simple straight line or it can be a shape that requires to remove the cut out trim during the manufacturing process. The opening cut may be shaped in a way that it allows to identify easily the position for insertion of the clip-on module, which allows easy insertion and that can hold the module securely in place after insertion.

In a preferable embodiment, the conductive pattern 101' extends symmetrically in a direction substantially parallel to the length of the substrate 1 and in a direction substantially perpendicular thereto, defining a plurality of independent electrical circuits, and wherein combinations of said electrical circuits are in electrical communication by selectively addressing at least two of the connection tracks with at least two corresponding terminals 33, or connection ports 33, of the clip-on data processing module 103. Preferably, the number of terminals 33 of the clip-on module 103 is at least the same as the number of connection tracks 1',2',3',4',5'. Preferably, at least two terminals or connection ports 33 of the clip-on data processing module 103 can be connected to a voltage source. The voltage source may be part of a conductive circuit that incorporates at least a portion of the conductive pattern 101'. Alternatively, the voltage source may be part of a circuit incorporated into the clip-on module 103 and hereby in contact to the terminals 33.

Various circuit design can be used for activation upon connecting the voltage source to connections ports 33 of the clip-on module 103, which in turn are connected to corresponding connection tracks 1',2',3',4',5'.

The selection of connection between connection tracks 1',2',3',4',5' and/or of the terminals 33 may be made electronically. By way of example, such an electronic selection can be achieved by controlling available terminals 33, or connection ports, 33 of the clip-on data processing module 103 via one or more switches. One part of said exemplary circuit may belong to the conductive pattern 101' whereas another part that comprises the voltage source and switches may be part of the clip-on module 103, or alternatively may be connected to the terminals, of the clip-on module 103.

Also, combinations of circuits defined by the conductive pattern 101' may be enabled by selectively addressing multiple combinations of at least pairs of connection tracks by multiple combinations of corresponding pairs of terminals 33, or connection ports,33. Moreover, by selectively addressing multiple combinations of said plurality of connection tracks with corresponding terminals of the clip-on data processing module 103, measurements of resistance, conductance, impedance and/or capacitance through the multiple electrical circuits defined by said conductive pattern 101' are also enabled.

In an embodiment, the conductive pattern 101' extends symmetrically in a direction substantially parallel to the length of the substrate 1 and in a direction substantially perpendicular thereto defining a plurality of independent electrical circuits, and wherein combinations of said electrical circuits are in electrical communication by selectively addressing at least two of the connection tracks 1', 2', 3', 4',5' with at least two corresponding terminals 33 or connection ports 33 of the clip-on data processing module 103, as explained before.

Moreover, the conductive pattern 101' may have a shape selected from straight lines and/or curved lines. Advantageously, said arrangements may circumvent some defects on the printing process placing the conductive pattern 101' onto the first surface 2 of the substrate 1.

The conductive pattern 101' is in the form of an electrically conductive ink as described previously and the application step comprises the printing thereof onto the backsheet. Moreover, the process generally comprises the step of detecting registered marks to trigger accurate cutting of the substrate 1 (or the assembled/laminated continuous absorbent article 100) into a plurality discrete absorbent articles.

In an embodiment, the sensing tracks 9 of the conductive pattern 101' may have a connected shape. These shapes may comprise, for example, solid or open rectangles and/or solid or open triangles and/or solid or open ellipses or a combination thereof. Preferably, sensing tracks 9 have a "T" shape with a height equal to the width of the sensing track and a width equal to 5 times the width of the sensing track.

Whatever the sensor tracks 101, or conductive pattern 101', arrangement, monitoring and detecting the presence of wetness at different zones of the core of the absorbent article 100 is enabled by measuring resistance, conductance, impedance and/or capacitance of multiple circuit configurations defined by the conductive pattern 101' and/or tracks 101 and selectively addressed by corresponding terminals 33, or connection ports 33, of the clip-on data processing module 103.

### SECOND SENSOR

The sensor system 102 according to the invention comprises a second sensor 112. Said second sensor 112 may be configured to measure temperature of or within the absorbent article 100.

According to an embodiment, the second sensor 112 can correspond to a temperature sensor that is comprised in the clip-on module 103. For example, an array of printed conductive tracks can be arranged on the substrate 1 of the absorbent article 100 and be in electrical communication with the second sensor 112 contained in the clip-on module 103. According to another embodiment, the second sensor 112 may be a temperature sensor arranged outside the clip-on module 103 that sends the temperature data directly to the control system 116.

The substrate 1 can further comprise at least one or a plurality of thermal sensor tracks 121. As illustrated on Fig. 1, the at least one or plurality of thermal sensor tracks 121 may comprise at least one central thermal sensor track 124 extending parallel to a length L of the substrate and parallel to a longitudinal axis y-y crossing a first end 5 and a second end 6 of the substrate 1. The thermal sensor tracks 121 may further comprise at least one or more, here two, side thermal sensor tracks 127, 128 preferably extending parallel to the central track and oppositely arranged such that the central track extends therebetween. The substrate 1 can further comprise additional side thermal sensor tracks extending outboard of said at least one or more side tracks 127,128. Having more thermal sensor tracks 121 enables to have a better temperature mapping of the absorbent article 100 and pinpointing which regions of the absorbent article 100 is being wetted.

At least one or a plurality of thermal sensor tracks 121 are in electrical communication with the second sensor 112. More precisely, the at least one central thermal sensor track 124 and/or said at least one or more side tracks 127,128 are in electrical communication with the second sensor 112. At least one of the central thermal sensor track 124 and said at least one or more side tracks 127,128 can be in electrical communication directly with at least one connecting port 33. The central thermal sensor track 124 and said at least one or more side tracks 127,128 can also be in electrical communication via one or more shortening elements 10 positioned proximal to said second end 6 and distal from said first end 5. In this embodiment, the flexible connection member 31 can comprise connection ports 33 at both free ends 32 of the clip-on module 103.

The one or plurality of thermal sensor tracks 121 can be arranged on the first surface 2 and/or on the second surface 3 of the absorbent article. It is possible to arrange the thermal sensor track 121 on the back sheet of the absorbent article 100. It is also possible to dispose the thermal sensor tracks 121 on the first surface 2 of the absorbent article 100, for example, by arranging at least one thermal sensor track 121 on the isolating layer 200.

The second sensor 112 can exploit different temperature probes, or detectors, including, and not limited to, Negative Temperature Coefficient temperature probes using thermistors, Resistance Temperature Detector temperature probes as mentioned above, pyroelectric detectors, or thermocouple temperature probes.

Preferably, the thermal sensor tracks 121 comprise an electrically conductive material, and are preferably printed thermal sensor tracks. In a preferred embodiment, but not limited to, the printed thermal sensor tracks 111 comprise, preferably consist of, a composite of conductive filler with polymer, and temperature sensing conductive materials such as, and not limited to, silver nanowire (AgNW) , carbon nanotubes (CNTs), reduced graphene oxide (rGO), and/or poly(3,4-ethylenedioxythiophene): poly(styrenesulfonate) (PEDOT:PSS). The printed thermal sensor tracks 121 can also comprise, preferably consist of, a carbon-based ink and/or a conductive polymer-based ink, preferably the carbon-based ink comprising a conductive compound selected from the group consisting of graphene, graphite, nano-carbon-tubes and mixtures thereof, preferably the conductive polymer-based ink comprising a conductive compound selected from the group consisting of polyacetylene, polypyrrole, polyaniline and copolymers thereof, more preferably selected from the group consisting of poly(pyrrole)s (PPY), polyanilines (PANI), poly(thiophene)s (PT), poly(p-phenylene sulfide) (PPS), poly(p-phenylene) (PPP), Poly(acetylene)s (PAC), Poly(p-phenylene vinylene) (PPV), poly(3,4-ethylenedioxythiophene) (PEDOT), and mixtures thereof, most preferred conductive polymer-based ink comprising poly(3,4-ethylenedioxythiophene) polystyrene sulfonate (PEDOT:PSS).

According to another embodiment, the second sensor 112 may exploit video monitoring using multispectral and/or thermal sensors and imaging to detect wetness/spill/soil/exudate events. In other words, the second sensor 112 can exploit techniques such as thermography for producing an image of invisible infrared light emitted by objects with the use of a camera 131, preferably a thermal imaging camera. Multispectral imaging is a superset of thermal imaging and may be defined as technique for capturing image data at specific frequencies across the electromagnetic spectrum. The multispectral and/or thermal imaging camera 131 is operable to produce an image showing the temperature differences of a surface. Images from the multispectral and/or thermal imaging camera 131 may then be interpreted to detect liquids and more specifically bodily fluids.

The second sensor 112 can be a component of the multispectral and/or thermal imaging camera 131 (as illustrated in Fig. 9), or the second sensor 112 can be a component of the clip-on data processing module 103 (as illustrated in Fig. 2). The invention is not limited to these illustrated embodiment, the second sensor 112 for example can be a component of another module arranged on the absorbent article 100, that is remote and distinct from the clip-on data processing module 103.

The multispectral and/or thermal imaging camera 131 can generate a chronological series of frames (e.g., as images). The multispectral and/or thermal imaging camera 131 can be analog or digital. The multispectral and/or thermal imaging camera 131 can capture a sequence of frames at a predetermined frame rate, such as six, eight, sixteen, twenty-four, or some other number of frames per second. The resolution of digital camera is usually defined by the number of pixels both horizontally and vertically (such as 640x480) or as a total number of pixels in the image (such as 1.4 mega pixels), while the resolution of analog video camera is typically defined by the number of television lines. Analog frames can be converted to digital frames by analog-to-digital conversion circuitry (e.g., as part of the computing system of the control system and/or the camera). The multispectral and/or thermal imaging camera 131 can have infrared illumination or night vision capabilities for operating in low light conditions. According to one embodiment, the camera 131 may include a thermographic camera (or thermal imaging camera) capable of producing a thermal image of objects using infrared, or other non-visible or visible spectral, radiation. A multispectral and/or thermal imaging camera 131 can be arranged stationary next to a bed, and continuously monitors the temperature level across the outer surface of the absorbent article 100. This stream of thermal images can be transmitted to the second sensor 112 or directly to the controller system 116. The stream of thermal images can be continuously processed by an image analysis system, which is either in the controller system 116 or in the user interface 118, which allows to translate changes of the temperature pattern into information on wetness/spill/soil/exudate events.

The second sensor 112 can be included as a component of the multispectral and/or thermal imaging camera 131 or as a component of the control system 116 or as a component of the clip-on module 103 as described above.

The multispectral and/or thermal imaging camera 131 can include optics 30. Optics 30 can include a lens, a filter, and/or other components for capturing and conditioning the light of the patient area. The multispectral and/or thermal imaging camera 131 can further include a sensor for converting light from the optics into electronic signals. Different types of sensors can be used depending on whether the multispectral and/or thermal imaging camera 131 is analog (e.g., generating analog video) or digital (e.g., generating discrete digital frames). The sensor can include a charge-coupled device (CCD), a complementary metal-oxide-semiconductor (CMOS), or a specialized thermal imaging focal plane array (FPA) image sensing device.

The multispectral and/or thermal imaging camera 131 can further include a processor and memory. The processor can perform various computing functions, such as those described herein or otherwise useful for operating the multispectral and/or thermal imaging camera 131. The processor can correspond with the second sensor 112 for example. The memory can be a non-transient computer readable storage medium (e.g., random access memory or flash) for storing program instructions and/or frames. For example, the processor can be configured to execute program instructions stored on the memory for controlling the camera 131 in converting visible or non-visible light from an area into digital signals, storing the digital signals on the memory as frame data, transferring the frame data to the controller system 116, and/or performing any other function with the second sensor 112. The processor may perform various signal conditioning and/or image processing on the frames. The processor may include a dedicated video processor for image processing. Although not illustrated, the multispectral and/or thermal imaging camera 131 can further include a network interface controller and a power supply. The camera 131 may include a user interface which can include user controls and/or an audible alarm.

Various camera devices and techniques can be employed to perform a scan of a patient area to collect information. Such techniques include infrared thermography (IRT), thermal imaging, and thermal video. For example, thermographic cameras may be used to detect radiation in the infrared range of the electromagnetic spectrum and produce thermal images of that radiation. Thermal images may include pixels with color, contrast, and/or intensities dependent on a range of infrared radiation emitted by all objects with a temperature above absolute zero. The amount of radiation emitted by an object increases with temperature and allows one to see variations in temperature. As such, warm objects (e.g., live human body and hot liquids) in a multispectral and/or thermal image stand out well against an environment that is cooler. As a result, thermal images are particularly useful for detecting the presence of bodily fluids.

According to a preferred embodiment, the multispectral and/or thermal imaging camera 131 is a stationary infrared dome camera. Such camera can easily be installed on the ceiling of a room and enable to monitor a plurality of patients simultaneously.

According to an embodiment, the multispectral and/or thermal imaging camera 131 can also be used as a motion sensor by detecting one or more zones of the absorbent article outline based on a pixel grouping. Various three dimensional scanning systems can generate point clouds. A point cloud can include a plurality of pixels grouped together, the group representative of the external surface of an object. Each pixel can have coordinates along X, Y, and Z axes. Surfaces can be interpolated between the pixels within the group to generate a three dimensional model of various objects in a scene.

An algorithm can compare pixel characteristics of a zone between sequential frames to determine whether the pixel characteristic of the zone has changed, the change indicative of either patient movement and/or liquid displacement. Patient movement and liquid displacement may be distinguished by features in the infrared, and other spectral planes, images such as thermal or spectral signature and size of moving pixel groups. A thermal signature may include the amount of heat, heat range, heat in certain areas, heat or spectral uniformity and heat or spectral stability of an object. A thermal image characterizes the intensity of the temperature associated with pixels. Spectral images characterize additional characteristics. Temperature can be used for motion detection by identifying changes in the temperature of a zone over time. The temperature of a zone may change over time (e.g., between sequential frames) because the temperature of the surfaces within the zone can change due to an apparition of bodily fluid (wetness/spill/soil/exudate event) within an image. In this embodiment, the use of a third sensor 114 acting as a motion sensor is not necessary. According to another embodiment, the multispectral and/or thermal imaging camera 131 has two functions, one detecting the temperature and another detecting the motion or positioning of or within the absorbent article 100.

### THIRD SENSOR

According to an embodiment, the sensor system 102 may comprises a third sensor 114. Said third sensor 114 may be configured to detect motion of the absorbent article 100.

The third sensor 114 or the motion sensor (also referred to as position sensor) comprises for example and not limited to, an accelerometer, a gyroscope or a multispectral and/or thermal imaging camera 131 using the pixel grouping as described above. The accelerometer and a gyroscope can be used for measuring linear and/or angular accelerations, the latter can be used to determine changes of the body posture of the wearer. In other words, the measurement of motion may refer to tracking a patient's position. Preferably, wetness/spill event data and the position data measured by the clip-on module 103 are combined for determination of the saturation level at a specific location of the absorbent article 100, typically determined respectively for a plurality of local areas of said absorbent article 100. An advantage of this arrangement is that it more accurately provides warnings via the user interface 118 to the care givers when the risk of leakage is high thus allowing them to intervene and limit additional cleaning time and costs. The motion sensor described herein is not particularly used to optimize the volume measurement, but rather to combine the position data with the resistive/impedance measured and the temperature data in order to determine a "local" saturation of the absorbent article, and thereafter preferably predict the risk of leakage in said localized area of the absorbent article.

### ASSEMBLY OF ABSORBENT ARTICLE AND SENSOR SYTEM

In an embodiment, the assembly of the absorbent article 100 with the sensor system 102 is arranged to measure and monitor the amount of wetness/soil/exudates across a surface covered by a plurality of sensor tracks 101, more specifically by the wetness sensing tracks 9, combined with the monitoring of temperature by one or a plurality of thermal sensor tracks 121, eventually combined with detection of the position of the subject (e.g. sitting, lying on back or side etc.) by a motion sensor comprised in the module 103 described herein above or by a multispectral and/or thermal imaging camera 131 as described above. The position information may then be used by the processor of the second sensor 112 or of the control system 116 to calculate an effect of said position (e.g. angled gravity effects) on the speed of saturation at a given area of the absorbent article and is compared and/or combined with sensed wetness signals received in said given area by the respective portion of the wetness sensing tracks 9 as well as the temperature signal measure on the thermal sensor tracks 121 or by the multispectral and/or thermal imaging camera 131. Preferably, the saturation level is determined for a plurality of individual and/or local areas within the absorbent article. This is advantageous over prior art systems that rather provide total saturation information, in that leakage risk may actually be high in a specific single region of the absorbent article even if the overall/total saturation of the absorbent article is far from being reached. In such instances, leakage may still occur and a warning to the care giver not provided, to the contrary the absorbent articles described herein advantageously monitor area-specific wetness information combined with position data in order to more effectively warn care givers when the risk of leakage is high such to prompt an intervention (e.g. rotate the patient to a new position or replace the absorbent article).

Since not only the volume inside an absorbent article defines the moment of leakage, but the position of a person's body over time also plays a fundamental role in determining the distribution of the liquid inside the absorbent article. The present disclosure addresses this with the combination of totally absorbed volume, number or wetness events as defined by the temperature spikes and more accurate position detection of a person's body (e.g. lying (on the belly/back/left side/right side), sitting or standing) over time determines when an absorbent article will most likely start leaking. The more accurate position detection as referred to, is for instance not only related to lying or sitting, but implying that a person is lying on a particular side of its body, or in case of sitting, that a person is sitting straight or under a certain angle. It has surprisingly been found that the particular sensor track and thermal sensor tracks arrangement described herein synergistically operates with the position sensing system to provide accurate determination of risk of leakage in use.

### CLIP-ON MODULE

In an embodiment, the absorbent article 100 comprises a removable clip-on data processing module 103 adapted to monitor and process resistance and/or capacitance data acquired from the wetness sensing tracks 9, said clip-on data processing module 103 being connectable to the at least one central track 4 and the at least two side tracks 7,8 via a slit 15 and/or pocket on the backsheet enabling an electrically conductive portion of said module 103 to directly come in electrical communication with said at least one central track 4 and said at least two side tracks 7,8, preferably the slit and/or pocket arranged to accommodate at least 50%, preferably at least 60%, more preferably at least 70%, even more preferably at least 80%, of the total surface area of the clip-on data processing module 103 therein. An advantage of this arrangement is that the module remains nicely secured to the absorbent article as well as being protected from accidental disengagement during movements of the subject/wearer.

The clip-on data processing module 103 can also be adapted to monitor and process temperature data acquired from the thermal sensor tracks 121, said clip-on data processing module 103 being connectable to the at least one central thermal sensor track 124 and eventually at least one thermal side tracks 127,128 via a slit 15 and/or pocket on the backsheet enabling an electrically conductive portion of said module 103 to directly come in electrical communication with said at least one central thermal sensor track 124 and eventually said at least one thermal side tracks 127,128

Preferably, the substrate 1 is positioned such that the first surface 2 faces the absorbent core, and preferably wherein the insulating layer 200 is joined to said substrate 1 such that a liquid impermeable seal is formed providing a barrier to body fluids expelled by a subject, when wearing the absorbent article, from coming into direct contact with the at least one central track 4 and the at least two side tracks 7,8. Preferably, said insulating layer 200 is adapted to prevent liquid from coming into contact with said central track 4 and said side tracks 7,8 as well as on the thermal sensor tracks 121 when they are printed on the insulating layer 200. It is highly preferred that at least a substantial portion of the wetness sensing tracks 9 remains exposed and not covered by said insulating layer 200.

In an embodiment, the insulating layer 200 comprises the one or more shortening elements 10, such that the central track 4, the side tracks 7,8, and the wetness sensing tracks 9 are in electrical communication via said shortening elements 10 only when said insulating layer 200 is joined to the substrate 1. An advantage of this arrangement is that a greater degree of flexibility is provided as well as simplifying the process of assembly for reasons that will be better explained in the process section herein.

In an embodiment, the insulating layer 200 adheres to the backsheet via a non-electrically-conductive adhesive and/or mechanical bonding, wherein the mechanical bonding is preferably selected from ultrasonic bonding, thermal bonding, and combinations thereof. An advantage of this arrangement is that noise and/or risk of compromising the wetness detection data is minimized.

In a preferred embodiment, the adhesive and/or mechanical bonding is comprised across a length and width of the insulating layer 200 in an effective amount such that bonding is achieved with the backsheet and a liquid impermeable seal is formed over the at least one central track 4 and the at least two side tracks 7,8, preferably wherein at a location proximal to the first end 5 of the substrate where the clip-on data processing module 103 is to be connected the adhesive is present only outboard of the at least two side tracks 7,8 and not therebetween such to form a pocket for receiving one or more electrically conducting connection ports 33 of the module 103.

In a preferred embodiment, the first end 5 of the substrate (and/or backsheet) corresponds to the front (or belly portion) of the absorbent article and the second end 6 corresponds to the back of the absorbent article.

As shown in Fig. 8 the at least a portion of the wetness sensing tracks 9 that may be alternatingly directly connected and disconnected to the respective side tracks 7,8 along the length L of the substrate 1 is preferably located at positions proximal to the front and/or back (and typically not a central portion positioned therebetween) of the absorbent article. An advantage is that improved wetness monitoring is achieved in the front and back regions of the absorbent article that, together with the sides thereof, is found to be most desirable when detecting risk of leakage.

In an embodiment, the backsheet comprises a slit 15 at a location proximal to the first end 5 of the substrate 1 where the clip-on data processing module 103 is to be connected, said slit being sized such to receive therethrough, typically only, the flexible connection member 31 typically comprising the connection ports 33 of the clip-on data processing module 103 and wherein, once said clip-on data processing module 103 is connected, said backsheet is interposed between the free end 32 of the flexible connection member 31 and the housing 30 of the clip-on data processing module 103 such that said free end 32 does not come in direct contact with said housing at a position proximal to said free end 32, preferably wherein the housing 30 is positioned on a garment facing side of the backsheet and the free end 32 of the flexible connection member 31 on a body facing side of the backsheet. An advantage of this arrangement is that a secure connection may be achieved between the module and the chassis of the article and in particular a very secure electrical connection to the sensor tracks is enabled, whilst it being protected from soiling by internal and/or external elements like dust, exudates, food and the like. This configuration is adapted for the embodiment in which the second sensor 112 is a multispectral and/or thermal imaging camera 131 or for the embodiment where the thermal sensor tracks 121 are arranged on the body facing side namely when the thermal sensor tracks 121 are printed unto the insulating layer 200.

As illustrated on Fig. 7, the clip-on data processing module 103 comprises a housing 30 having a U-shaped cross section with two flexible connection members 31 corresponding to the branches as illustrated in Fig. 7. The flexible connection members 31 each comprises connection ports 33. In order words, there are connection ports 33 arranged on the flexible connection member 31 on garment facing side of the backsheet and on a body facing side of the backsheet. This configuration is adapted for the embodiment in which the thermal sensor tracks 121 are arranged on the garment facing side. This way, the first set of connection ports 33 arranged on the body facing side of the backsheet establish the electrical connection between the clip-on data processing module 103 and the sensor tracks 101 and the second set of connections ports 33 arranged on the garment facing side of the backsheet establish the electrical connection between the clip-on data processing module 103 and the thermal sensor tracks 121.

### THE PROCESS

The disclosure herein further contemplates a process of making an absorbent article comprising the steps of: providing a liquid impermeable backsheet and applying a plurality of sensor tracks and optionally thermal sensor tracks as described herein; providing an insulating layer 200 having a width w, taken along an axis perpendicular to the longitudinal axis y-y, being less than a width W of said backsheet, and typically applying one or more shortening elements 10 thereto, optionally further applying one or more secondary shortening elements 11 thereto; adhering said insulating layer 200 to said backsheet, said insulating layer being sized and positioned to cover the at least one central track 4 and the at least two side tracks 7,8, to provide a laminated substrate; providing an absorbent core comprising absorbent material; providing a liquid permeable topsheet; and sandwiching the absorbent core between said backsheet and said topsheet. Preferably, the sensor tracks are in the form of an electrically conductive ink and the application step comprises the printing thereof onto the backsheet, preferably wherein the shortening elements 10 and the secondary shortening elements 11 are in the form of an electrically conductive ink and the application step comprises the printing thereof onto the insulating layer 200 or backsheet.

In an embodiment, the layers referred to herein above (i.e. at least the backsheet and the insulating layer) are in the form of continuous webs and/or films that are first printed with conductive ink in the pattern described above (sensor tracks and shortening elements respectively) prior to being laminated together and subsequently cut into individual absorbent articles.

In an embodiment, the insulating layer 200 is adhered to the backsheet by a non-electrically-conductive adhesive and/or mechanical bonding, wherein the mechanical bonding is preferably selected from ultrasonic bonding, thermal bonding, and combinations thereof. In a preferred embodiment, the adhesive and/or mechanical bonding is applied across a length and width of the insulating layer 200 in an effective amount such that bonding is achieved with the backsheet and a liquid impermeable seal is formed providing a barrier to exudates expelled by a subject, when wearing the absorbent article 100, from coming into direct contact with the at least one central track 4 and the at least two side tracks 7,8, preferably wherein at a location proximal to the first end 5 of the backsheet where the clip-on data processing module 103 is to be connected the adhesive is present only outboard of the at least two side tracks 7,8 and not therebetween such to form a pocket for receiving an electrically conducting portion of said module 103.

### THE INCONTINENCE MANAGEMENT SYSTEM

In accordance to an aspect of the disclosure, an incontinence management system is provided for managing raw data generated by the modules described herein in cooperation with the sensor tracks, the thermal sensor tracks and/or the multispectral and/or thermal imaging camera 131 described above and located on a substrate of respective absorbent articles, and adapted to process the raw data to processed data, transfer the raw data and/or the processed data over a network, and link the raw data and/or the processed data with person data of a given patient or individual. The management system may comprise a module 103 comprising a transmitter as described herein above for delivering the raw data, a cloud server for processing the raw data to processed data, preferably by applying a mathematical logarithmic model, and a client application or graphical user interface for linking the raw data and the processed data with person data. The management system may comprise a module 103 comprising a transmitter as described herein above for delivering the raw data to the controller system 116 described herein, a processor for processing the raw data to processed data, preferably by applying a mathematical logarithmic model, and a client application or graphical user interface 118 for linking the raw data and the processed data with person data. The management system may optionally further comprise one or more docking stations for initializing the modules 103 for first time use, the latter may also be done via a portable device such as a smart phone and/or tablet.

In a preferred embodiment, the network herein is a wireless network being a sub-Ghz wireless network (i.e. having data rates of from 20 kbit/s (868 MHz band) to 250 kbit/s (2.4 GHz band) such as Zigbee) which is preferred over wifi that typically operates at higher data rates (from 2.4 Ghz to 5 Ghz). An advantage of this arrangement is to provide a large broadcasting distance, low energy consumption and to remain independent from any existing wifi networks that for example may be in place at a given institution or elderly home, so that the system described herein does not need to share bandwidth with other applications thus improving reliability and consistency of data acquisition and processing.

In an embodiment, the disclosure further contemplates an incontinence management system for managing the measured or captured data (by the absorbent articles and modules described herein), thereby using a network wherein the data can be transmitted, for example anonymized patient data (typically of relevance for instance in a nursing home or residential care institution and the like). The measured data or sensor data as used herein is also referred to as raw data.

The clip-on modules described herein may comprise a unique identifier via its media access control (MAC) address which may be linked to an individual or patient. Preferably, the unique identifier may be comprised in the form of indicia (e.g. a OR-code) applied to an external surface of the clip-on modules that may be scanned by a linking device (such as smart phone or tablet) for linking a given module to a given individual and/or patient. The linking may alternatively be done manually by inputting the identifier number of a given module to the linking device by typing the same.

In an embodiment, a plurality of modules as described herein are connected to the cloud where the sensor data is linked with an individual or patient, preferably anonymously via numerical identifiers randomly generated for each individual or patient. Preferably, the raw data is processed within the cloud wherein one or more mathematical models may be applied to further compute and predict a plurality of wetness status, pathological status, and/or physiological status for each individual/patient. Referring to the application within a nursing home for instance, a linking dock may be provided locally in the nursing home, and acting as network access point and server.

In an embodiment the network is a wireless network, e.g. a local wireless network such as wireless local area network (WLAN) (typically using WiFi^{™}) and/or Bluetooth^{™} for the wireless connection. Most preferred wireless network however remains Zigbee as described above, and herein after embodiments that specifically refer to wifi can be, and are preferably implemented, with Zigbee. Regarding the use of a unique identifier, a service set identifier (SSID) is then for instance used over the WLAN and may be provided by the linking dock. The modules may be linked with the linking dock prior to each module being installed. Moreover, the linking dock may provide an initialization and/or setting startup/installation of the module to pre-set and/or activate it for use, within this initialization the module is provided with the unique anonymous individual/patient number, stores this number and relates this number (typically through a database) with real patient identification such as a person's name for example. A client application may further be part of the network, and may be directly linked with the linking dock, and hence generally a nursing home patient dashboard. Via the client application, the linking dock or nursing home dashboard is able to indicate which module is linked with a particular patient, whereas the real individual/patient identification is stored in a separate database that is linked with the linking dock. Real patient information can be displayed by the client application such as for example a tablet or smart phone. Such information includes for instance not only a real person's identification, but also processed data or computed information from the cloud or the controller system 116.

In an embodiment, the measured data is captured by means of the module described herein (typically by receiving and processing resistive and/or capacitance signals through the plurality of sensor tracks comprised in a substrate of the absorbent article as described herein). The measured data (comprising raw data) is subsequently transferred to the cloud by means of a wireless data transfer (typically via the transmitter present the modules described herein above). Preferably, the measured data is pre-processed by the processor comprised in the module and subsequently transmitted to the cloud for application of the mathematical model used to predict for example the saturation level of the absorbent article and/or the risk of leakage. An advantage of the latter arrangement is that the heavy signal processing and computing is decoupled from the actual hardware connected to the absorbent article, thus allowing reduced power consumption and device complexity which in turns makes for simpler large scale manufacturing as well as reduced module size for comfort of wear and handling.

In an embodiment, the modules are distributed and installed to the absorbent articles (by connecting to the chassis thereof as described herein) of the corresponding individuals or patients. Each module may comprise a visual identification number, e.g. between 1 and 999 typically displayed on a portion of the display described herein above. This may be useful when replacing one module with another for servicing, cleaning and the like for a given patient, for example when replacing a module the new module may acquire the identification number of the replaced module either manually by inputting it directly to the module or preferably automatically via the client application or graphical user interface (GUI). In parallel, the GUI may also inform the cloud server that the unique identifier is now linked to the newly replaced module.

In an embodiment, the raw data is communicated to the cloud server, which recognizes the identifier of the module and links this data to a unique anonymous identifier of the patient. The cloud server processes, computes or calculates useful information from this raw data and makes such information accessible in graphical representation for different end-users or care givers using a GUI or client application.

In an embodiment, the raw data is communicated to the controller system 116, which recognizes the identifier of the module and links this data to a unique anonymous identifier of the patient. The controller system 116 processes, computes or calculates useful information from this raw data and makes such information accessible in graphical representation for different end-users or care givers using a GUI or client application.

Preferably, the end-users such as for instance care givers or other nursing home personnel can connect with the cloud or the user interface 118 and extract e.g. the needed information for their typical department. The data from the cloud or controller system 116 is still anonymous and the unique anonymous identifiers have to be replaced with real names and information. The client application or GUI accesses the linking dock server such that the useful information or other cloud data is linked to a real name of an individual or patient. Thus, the module delivers raw data linked to a unique identifier, such as e.g. a MAC address. The cloud server or controller system 116 processes such data and links such unique identifier to a unique person or patient number. The processed data can now be accessed by a client application. The client application replaces the unique person or patient number with a real person or patient identification, including for example information regarding room number, age, and name.

It is supposed that the present invention is not restricted to any form of realization described previously and that some modifications can be added to the presented example of fabrication without reappraisal of the appended claims.

## Claims

1. A sensor system (102) configured for detecting a property of or within an absorbent article (100), comprising:
- a first sensor (110) configured to measure a first physical parameter, said first sensor (110) being configured to detect a change in condition of or within the absorbent article (100);
- a second sensor (112) configured to measure a second physical parameter, said second sensor (112) being configured to detect a change in condition of or within the absorbent article (100);
wherein the first and second physical parameters are different from one another, wherein the first sensor (110) is configured to measure electrical resistance or electrical conductance and the second sensor (112) is configured to measure temperature and wherein the second sensor (112) is a multispectral and/or thermal imaging camera (131).

2. Sensor system (102) according to claim 1, wherein the second sensor (112) is distinct and remote from the first sensor (110).

3. Sensor system (102) according to any of the claims 1 or 2, wherein the second sensor (112) is linked to a temperature probe.

4. Sensor system (102) according to any of the claims 1 to 3, wherein the sensor system (102) comprises a third sensor (114) configured to measure a third physical parameter, said third sensor (114) being configured to detect a change in condition of or within the absorbent article; wherein the first, second and third physical parameters are different from one another.

5. Sensor system (102) according to any of the claims 1 to 3, wherein the sensor system (102) monitors the temperature and electrical resistance, or electrical conductance, behaviour of or within an absorbent article (100) by measuring the temperature and electrical resistance or electrical conductance values and plotting said values against time.

6. Sensor system (102) according to any of the preceding claims, wherein the sensor system (102) further comprises a control system (116) that manages the output data of first, second and/or third sensors (110,112,114), said sensor system (102) further comprises a user interface (118) that manages the output data of the control system (116).

7. Sensor system (102) according to claim 6, wherein the control system (116) manages the output data of the second sensor (112), and if a rise in temperature is detected, the control system (116) activates the first sensor (110) in order to measure electrical resistance, or electrical conductance, of or within the absorbent article (100).

8. Assembly of an absorbent article (100) and a sensor system (102) according to any of the claims 1 to 7.

9. Assembly according to claim 8, wherein the absorbent article comprises a substrate (1) suitable for incorporation into an absorbent article (100) for automatic detection of wetness events therein and/or risk of exudate leakage therefrom, the substrate comprising a first surface (2) capable of being arranged proximal to a body facing side of the absorbent article (100) and a second surface (3) opposite said first surface (2) and capable of being arranged proximal to a garment facing side of said absorbent article (100), said substrate (1) comprising a plurality of sensor tracks (101) disposed on said first surface (2) and said sensor tracks (101) comprising: at least one central track (4) extending parallel to a length L of the substrate and parallel to a longitudinal axis (y-y) crossing a first end (5) and a second end (6) of the substrate (1); at least one or more side tracks (7,8) extending parallel to the central track (4) and oppositely arranged such that the central track (4) extends therebetween; and wetness sensing tracks (9) extending outboard of said at least one or more side tracks (7,8), wherein said central track (4), said at least one or more side tracks (7,8), and said wetness sensing tracks (9) are in electrical communication via one or more shortening elements (10) positioned proximal to said second end (6) and distal from said first end (5), and wherein the substrate (1) is connectable to a clip-on data processing module (103) at a position proximal to said first end (5) and distal from said shortening elements (10) such to form a closed electrical circuit, typically for measuring electrical resistance, electrical conductance, impedance and/or capacitance therethrough.

10. Assembly according to claim 8, wherein the absorbent article comprises a substrate (1) suitable for incorporation into an absorbent article (100) for monitoring and detecting the presence of wetness therein and/or risk of exudate leakage therefrom, comprising a conductive pattern (101') disposed on a first surface (2) capable of being arranged proximal to a body facing side of the absorbent article (100), wherein said conductive pattern (101') can be brought in electrical communication with a clip-on data processing module (103), said conductive pattern (101') comprising a plurality of connection tracks (1',2',3',4',5'); and a plurality of sensing tracks (9) connected to said connection tracks (1',2',3',4',5'), **characterized in that** the conductive pattern (101') is configured in a manner that addressing multiple combinations of said plurality of connection tracks (1',2',3',4',5') with corresponding connection ports (33) of the clip-on data processing module (103) result in multiple electrical circuit configurations for measuring resistance, conductance, impedance and/or capacitance therethrough.

11. Assembly according to claim 9 or 10, wherein said substrate (1) further comprises a plurality of thermal sensor tracks (121) disposed on said second surface (3) and said thermal sensor tracks (121) comprising: at least one central thermal sensor track (124) extending parallel to a length L of the substrate and parallel to a longitudinal axis (y-y) crossing a first end (5) and a second end (6) of the substrate (1); and wherein the substrate (1) is connectable to a clip-on data processing module (103) at a position proximal to said first end (5) for measuring temperature therethrough.

12. Assembly according to claim 9 to 11, wherein the absorbent article further comprising a removable clip-on data processing module (103) adapted to monitor and process electrical resistance and/or temperature data acquired from the wetness sensing tracks (9) and/or thermal sensor tracks (121), said clip-on data processing module (103) is in electrical communication with the conductive pattern (101') when connected at a position proximal to a first end (5) of the substrate (1) such to form at least one closed electrical circuit or said clip-on data processing module (103) being connectable to the at least one central track (4) and the at least two side tracks (7,8) via a slit and/or pocket on the backsheet enabling an electrically conductive portion of said module (103) to directly come in electrical communication with said at least one central track (4) and said at least two side tracks (7,8), preferably the slit and/or pocket arranged to accommodate at least 50% of the total surface area of the clip on data processing module therein.

13. Assembly according to claim 12, wherein the clip-on data processing module (103) comprises a housing (30) having a U-shaped cross-section, the housing having two flexible connection member (31), the substrate (1) extending between the flexible connection members (31), each flexible connection member (31) comprising connection ports (33) to establish electrical connections between the sensor tracks (101) or conductive pattern (101') and/or thermal sensor tracks (121) and the clip-on data processing module (103).

## Patentansprüche

1. Sensorsystem (102), das zum Erkennen einer Eigenschaft eines oder innerhalb eines absorbierenden Artikels (100) konfiguriert ist, umfassend:
- einen ersten Sensor (110), der zum Messen eines ersten physikalischen Parameters konfiguriert ist, wobei der erste Sensor (110) zum Erkennen einer Zustandsänderung des oder innerhalb des absorbierenden Artikels (100) konfiguriert ist;
- einen zweiten Sensor (112), der zum Messen eines zweiten physikalischen Parameters konfiguriert ist, wobei der zweite Sensor (112) zum Erkennen einer Zustandsänderung des oder innerhalb des absorbierenden Artikels (100) konfiguriert ist;
wobei der erste und der zweite physikalische Parameter voneinander verschieden sind, wobei der erste Sensor (110) dazu konfiguriert ist, einen elektrischen Widerstand oder eine elektrische Leitfähigkeit zu messen und der zweite Sensor (112) dazu konfiguriert ist, eine Temperatur zu messen, und wobei der zweite Sensor (112) eine multispektrale und/oder Wärmebildkamera (131) ist.

2. Sensorsystem (102) nach Anspruch 1, wobei der zweite Sensor (112) unterschiedlich und entfernt vom ersten Sensor (110) ist.

3. Sensorsystem (102) nach einem der Ansprüche 1 oder 2, wobei der zweite Sensor (112) mit einem Temperaturfühler verknüpft ist.

4. Sensorsystem (102) nach einem der Ansprüche 1 bis 3, wobei das Sensorsystem (102) einen dritten Sensor (114) umfasst, der zum Messen eines dritten physikalischen Parameters konfiguriert ist, wobei der dritte Sensor (114) zum Erkennen einer Zustandsänderung des oder innerhalb des absorbierenden Artikels konfiguriert ist; wobei der erste, der zweite und der dritte physikalische Parameter voneinander verschieden sind.

5. Sensorsystem (102) nach einem der Ansprüche 1 bis 3, wobei das Sensorsystem (102) die Temperatur und den elektrischen Widerstand oder die elektrische Leitfähigkeit sowie das Verhalten eines oder innerhalb eines absorbierenden Artikels (100) überwacht, indem es die Werte für Temperatur und elektrischen Widerstand oder elektrische Leitfähigkeit misst und diese Werte im zeitlichen Verlauf aufzeichnet.

6. Sensorsystem (102) nach einem der vorstehenden Ansprüche, wobei das Sensorsystem (102) weiter ein Steuersystem (116) umfasst, das die Ausgabedaten des ersten, zweiten und/oder dritten Sensors (110, 112, 114) verwaltet, wobei das Sensorsystem (102) weiter eine Benutzerschnittstelle (118) umfasst, die die Ausgabedaten des Steuersystems (116) verwaltet.

7. Sensorsystem (102) nach Anspruch 6, wobei das Steuersystem (116) die Ausgabedaten des zweiten Sensors (112) verwaltet, und wenn ein Temperaturanstieg erkannt wird, das Steuersystem (116) den ersten Sensor (110) aktiviert, um den elektrischen Widerstand oder die elektrische Leitfähigkeit des oder innerhalb des absorbierenden Artikels (100) zu messen.

8. Anordnung aus einem absorbierenden Artikel (100) und einem Sensorsystem (102) nach einem der Ansprüche 1 bis 7.

9. Anordnung nach Anspruch 8, wobei der absorbierende Artikel ein Substrat (1) umfasst, das für die Eingliederung in einen absorbierenden Artikel (100) zur automatischen Erkennung von Nässeereignissen darin und/oder des Risikos eines Exsudataustritts daraus geeignet ist, wobei das Substrat eine erste Oberfläche (2), die proximal zu einer dem Körper zugewandten Seite des absorbierenden Artikels (100) angeordnet werden kann, und eine zweite Oberfläche (3) gegenüber der ersten Oberfläche (2) umfasst, die proximal zu einer der Kleidung zugewandten Seite des absorbierenden Artikels (100) angeordnet werden kann, wobei das Substrat (1) eine Vielzahl von Sensorbahnen (101) umfasst, die auf der ersten Oberfläche (2) angeordnet sind, und wobei die Sensorbahnen (101) Folgendes umfassen: mindestens eine zentrale Bahn (4), die sich parallel zu einer Länge L des Substrats und parallel zu einer Längsachse (y-y) erstreckt, die ein erstes Ende (5) und ein zweites Ende (6) des Substrats (1) kreuzt; mindestens eine oder mehrere Seitenbahnen (7, 8), die sich parallel zur zentralen Bahn (4) erstrecken und gegenüberliegend angeordnet sind, sodass sich die zentrale Bahn (4) dazwischen erstreckt; und Nässesensorbahnen (9), die sich außerhalb der mindestens einen oder mehreren Seitenbahnen (7, 8) erstrecken, wobei die zentrale Bahn (4), die mindestens eine oder mehrere Seitenbahnen (7, 8) und die Nässesensorbahnen (9) über ein oder mehrere Verkürzungselemente (10), die proximal zum zweiten Ende (6) und distal zum ersten Ende (5) positioniert sind, in elektrischer Verbindung stehen, und wobei das Substrat (1) an einer Position proximal zum ersten Ende (5) und distal zu den Verkürzungselementen (10) mit einem aufsteckbaren Datenverarbeitungsmodul (103) verbindbar ist, sodass ein geschlossener elektrischer Schaltkreis gebildet wird, typischerweise zum Messen von elektrischem Widerstand, elektrischer Leitfähigkeit, Impedanz und/oder Kapazität dadurch.

10. Anordnung nach Anspruch 8, wobei der absorbierende Artikel ein Substrat (1) umfasst, das für die Eingliederung in einen absorbierenden Artikel (100) geeignet ist, um das Vorhandensein von Nässe darin und/oder des Risikos eines Exsudataustritts daraus zu überwachen und zu erkennen, umfassend ein leitfähiges Muster (101'), das auf einer ersten Oberfläche (2) angeordnet ist, die proximal zu einer dem Körper zugewandten Seite des absorbierenden Artikels (100) angeordnet werden kann, wobei das leitfähige Muster (101') in elektrische Verbindung mit einem aufsteckbaren Datenverarbeitungsmodul (103) gebracht werden kann, wobei das leitfähige Muster (101') eine Vielzahl von Verbindungsbahnen (1',2',3',4',5') umfasst; und eine Vielzahl von Sensorbahnen (9), die mit den Verbindungsbahnen (1',2',3',4',5') verbunden sind, **dadurch gekennzeichnet, dass** das leitfähige Muster (101') derart konfiguriert ist, dass das Adressieren mehrerer Kombinationen der Vielzahl von Verbindungsbahnen (1',2',3',4',5') mit entsprechenden Verbindungsanschlüssen (33) des aufsteckbaren Datenverarbeitungsmoduls (103) zu mehreren elektrischen Schaltungskonfigurationen zum Messen von Widerstand, Leitfähigkeit, Impedanz und/oder Kapazität dadurch führt.

11. Anordnung nach Anspruch 9 oder 10, wobei das Substrat (1) weiter eine Vielzahl von Wärmesensorbahnen (121) umfasst, die auf der zweiten Oberfläche (3) angeordnet sind, und wobei die Wärmesensorbahnen (121) mindestens eine zentrale Wärmesensorbahn (124) umfassen, die sich parallel zu einer Länge L des Substrats und parallel zu einer Längsachse (y-y) erstreckt, die ein erstes Ende (5) und ein zweites Ende (6) des Substrats (1) kreuzt; und wobei das Substrat (1) an einer Position proximal zum ersten Ende (5) mit einem aufsteckbaren Datenverarbeitungsmodul (103) zum Messen der Temperatur dadurch verbindbar ist.

12. Anordnung nach Anspruch 9 bis 11, wobei der absorbierende Artikel weiter ein abnehmbares, aufsteckbares Datenverarbeitungsmodul (103) umfasst, das zur Überwachung und Verarbeitung von Daten über den elektrischen Widerstand und/oder die Temperatur angepasst ist, die von den Nässesensorbahnen (9) und/oder thermischen Sensorbahnen (121) erhalten werden, wobei das aufsteckbare Datenverarbeitungsmodul (103) in elektrischer Verbindung mit dem leitfähigen Muster (101') steht, wenn es an einer Position proximal zu einem ersten Ende (5) des Substrats (1) verbunden wird, sodass mindestens ein geschlossener elektrischer Schaltkreis gebildet wird, oder wobei das aufsteckbare Datenverarbeitungsmodul (103) mit der mindestens einen zentralen Bahn (4) und den mindestens zwei Seitenbahnen (7,8) über einen Schlitz und/oder eine Tasche auf der Rückseite verbindbar ist, wodurch ermöglicht wird, dass ein elektrisch leitendes Teil des Moduls (103) direkt mit der mindestens einen zentralen Bahn (4) und den mindestens zwei Seitenbahnen (7,8) in elektrische Verbindung tritt, wobei der Schlitz und/oder die Tasche vorzugsweise angeordnet ist, um mindestens 50 % der Gesamtoberfläche des aufsteckbaren Datenverarbeitungsmoduls aufzunehmen.

13. Anordnung nach Anspruch 12, wobei das aufsteckbare Datenverarbeitungsmodul (103) ein Gehäuse (30) mit U-förmigem Querschnitt umfasst, wobei das Gehäuse zwei flexible Verbindungselemente (31) aufweist, wobei sich das Substrat (1) zwischen den flexiblen Verbindungselementen (31) erstreckt, wobei jedes flexible Verbindungselement (31) Verbindungsanschlüsse (33) umfasst, um elektrische Verbindungen zwischen den Sensorbahnen (101) oder dem leitfähigen Muster (101') und/oder Wärmesensorbahnen (121) und dem aufsteckbaren Datenverarbeitungsmodul (103) herzustellen.

## Revendications

1. Système de capteurs (102) configuré pour détecter une propriété d'un article absorbant (100) ou à l'intérieur de celui-ci, comprenant :
- un premier capteur (110) configuré pour mesurer un premier paramètre physique, ledit premier capteur (110) étant configuré pour détecter un changement d'état de l'article absorbant (100) ou à l'intérieur de celui-ci ;
- un deuxième capteur (112) configuré pour mesurer un deuxième paramètre physique, ledit deuxième capteur (112) étant configuré pour détecter un changement d'état de l'article absorbant (100) ou à l'intérieur de celui-ci ;
dans lequel les premier et deuxième paramètres physiques sont différents l'un de l'autre, dans lequel le premier capteur (110) est configuré pour mesurer une résistance électrique ou une conductance électrique et le deuxième capteur (112) est configuré pour mesurer une température et dans lequel le deuxième capteur (112) est une caméra d'imagerie multispectrale et/ou thermique (131).

2. Système de capteurs (102) selon la revendication 1, dans lequel le deuxième capteur (112) est distinct et éloigné du premier capteur (110).

3. Système de capteurs (102) selon l'une quelconque des revendications 1 ou 2, dans lequel le deuxième capteur (112) est lié à une sonde de température.

4. Système de capteurs (102) selon l'une quelconque des revendications 1 à 3, dans lequel le système de capteurs (102) comprend un troisième capteur (114) configuré pour mesurer un troisième paramètre physique, ledit troisième capteur (114) étant configuré pour détecter un changement d'état de l'article absorbant ou à l'intérieur de celui-ci ; dans lequel les premier, deuxième et troisième paramètres physiques sont différents les uns des autres.

5. Système de capteurs (102) selon l'une quelconque des revendications 1 à 3, dans lequel le système de capteurs (102) surveille la température et la résistance électrique, ou la conductance électrique, le comportement d'un article absorbant (100) ou à l'intérieur de celui-ci, en mesurant les valeurs de température et de résistance électrique ou de conductance électrique et en traçant lesdites valeurs en fonction du temps.

6. Système de capteurs (102) selon l'une quelconque des revendications précédentes, dans lequel le système de capteurs (102) comprend en outre un système de commande (116) qui gère les données de sortie des premier, deuxième et/ou troisième capteurs (110, 112, 114), ledit système de capteurs (102) comprend en outre une interface utilisateur (118) qui gère les données de sortie du système de commande (116).

7. Système de capteurs (102) selon la revendication 6, dans lequel le système de commande (116) gère les données de sortie du deuxième capteur (112) et, si une augmentation de température est détectée, le système de commande (116) active le premier capteur (110) afin de mesurer la résistance électrique, ou la conductance électrique, de l'article absorbant (100) ou à l'intérieur de celui-ci.

8. Ensemble d'un article absorbant (100) et d'un système de capteurs (102) selon l'une quelconque des revendications 1 à 7.

9. Ensemble selon la revendication 8, dans lequel l'article absorbant comprend un substrat (1) approprié pour une incorporation dans un article absorbant (100) pour une détection automatique d'événements d'humidité à l'intérieur de celui-ci et/ou un risque de fuite d'exsudat à partir de celui-ci, le substrat comprenant une première surface (2) qui peut être agencée à proximité d'un côté faisant face au corps de l'article absorbant (100), et une seconde surface (3) opposée à ladite première surface (2) et qui peut être agencée à proximité d'un côté faisant face à un vêtement dudit article absorbant (100), ledit substrat (1) comprenant une pluralité de pistes de capteur (101) disposées sur ladite première surface (2) et lesdites pistes de capteur (101) comprenant : au moins une piste centrale (4) s'étendant parallèlement à une longueur L du substrat et parallèlement à un axe longitudinal (y-y) traversant une première extrémité (5) et une seconde extrémité (6) du substrat (1) ; au moins une ou plusieurs pistes latérales (7, 8) s'étendant parallèlement à la piste centrale (4) et agencées de manière opposée de telle sorte que la piste centrale (4) s'étende entre celles-ci ; et des pistes de détection d'humidité (9) s'étendant à l'extérieur desdites au moins une ou plusieurs pistes latérales (7, 8), dans lequel ladite piste centrale (4), lesdites au moins une ou plusieurs pistes latérales (7, 8) et lesdites pistes de détection d'humidité (9) sont en communication électrique par le biais d'un ou de plusieurs éléments de raccourcissement (10) positionnés à proximité de ladite seconde extrémité (6) et distaux de ladite première extrémité (5), et dans lequel le substrat (1) peut être connecté à un module de traitement de données à clipser (103) à une position proximale par rapport à ladite première extrémité (5) et distale par rapport auxdits éléments de raccourcissement (10) de manière à former un circuit électrique fermé, typiquement pour mesurer une résistance électrique, une conductance électrique, une impédance et/ou une capacité à travers celui-ci.

10. Ensemble selon la revendication 8, dans lequel l'article absorbant comprend un substrat (1) approprié pour une incorporation dans un article absorbant (100) pour surveiller et détecter la présence d'humidité à l'intérieur de celui-ci et/ou un risque de fuite d'exsudat à partir de celui-ci, comprenant un motif conducteur (101') disposé sur une première surface (2) qui peut être agencée à proximité d'un côté faisant face au corps de l'article absorbant (100), dans lequel ledit motif conducteur (101') peut être mis en communication électrique avec un module de traitement de données à clipser (103), ledit motif conducteur (101') comprenant une pluralité de pistes de connexion (1', 2', 3', 4', 5') ; et une pluralité de pistes de détection (9) connectées auxdites pistes de connexion (1', 2', 3', 4', 5'), **caractérisé en ce que** le motif conducteur (101') est configuré de manière que le traitement de multiples combinaisons de ladite pluralité de pistes de connexion (1', 2', 3', 4', 5') avec des ports de connexion correspondants (33) du module de traitement de données à clipser (103) aient pour résultat de multiples configurations de circuit électrique pour mesurer une résistance, une conductance, une impédance et/ou une capacité à travers celui-ci.

11. Ensemble selon la revendication 9 ou 10, dans lequel ledit substrat (1) comprend en outre une pluralité de pistes de capteur thermique (121) disposées sur ladite seconde surface (3) et lesdites pistes de capteur thermique (121) comprenant : au moins une piste de capteur thermique centrale (124) s'étendant parallèlement à une longueur L du substrat et parallèlement à un axe longitudinal (y-y) traversant une première extrémité (5) et une seconde extrémité (6) du substrat (1) ; et dans lequel le substrat (1) peut être connecté à un module de traitement de données à clipser (103) à une position à proximité de ladite première extrémité (5) pour mesurer une température à travers celui-ci.

12. Ensemble selon les revendications 9 à 11, dans lequel l'article absorbant comprend en outre un module de traitement de données à clipser amovible (103) adapté pour surveiller et traiter des données de résistance électrique et/ou de température acquises à partir des pistes de détection d'humidité (9) et/ou des pistes de capteur thermique (121), ledit module de traitement de données à clipser (103) est en communication électrique avec le motif conducteur (101') lorsqu'il est connecté à une position à proximité d'une première extrémité (5) du substrat (1) de sorte à former au moins un circuit électrique fermé ou ledit module de traitement de données à clipser (103) qui peut être connecté à la au moins une piste centrale (4) et aux au moins deux pistes latérales (7, 8) par le biais d'une fente et/ou d'une poche sur la feuille de fond permettant à une partie électroconductrice dudit module (103) d'entrer directement en communication électrique avec ladite au moins une piste centrale (4) et lesdites au moins deux pistes latérales (7, 8), de préférence la fente et/ou la poche étant agencées pour accueillir au moins 50 % de la surface totale du module de traitement de données à clipser à l'intérieur de celle-ci.

13. Ensemble selon la revendication 12, dans lequel le module de traitement de données à clipser (103) comprend un boîtier (30) présentant une section transversale en forme de U, le boîtier présentant deux éléments de connexion flexibles (31), le substrat (1) s'étendant entre les éléments de connexion flexibles (31), chaque élément de connexion flexible (31) comprenant des ports de connexion (33) pour établir des connexions électriques entre les pistes de capteur (101) ou le motif conducteur (101') et/ou des pistes de capteur thermique (121) et le module de traitement de données à clipser (103).
